Europäisches Patentamt

European Patent Office

Office européen des brevets

(19)

(11) **EP 1 068 226 B1**

(12) **FASCICULE DE BREVET EUROPEEN**

(45) Date de publication et mention
de la délivrance du brevet:
**20.04.2005 Bulletin 2005/16**

(21) Numéro de dépôt: **99911884.7**

(22) Date de dépôt: **06.04.1999**

(51) Int Cl.⁷: **C07K 14/025**, C07K 14/16,
A61K 39/295, A61K 47/48

(86) Numéro de dépôt international:
**PCT/FR1999/000792**

(87) Numéro de publication internationale:
**WO 1999/051630 (14.10.1999 Gazette 1999/41)**

(54) **LIPOPEPTIDES INDUCTEURS DE CYTOTOXICITE T LYMPHOCYTAIRE PORTANT AU MOINS UN EPITOPE T AUXILIAIRE, ET LEURS UTILISATIONS POUR LA VACCINATION**

**LIPOPEPTIDE, DIE EINE ZYTOTOXISCHE T-LYMPHOZYTENANTWORT INDUZIEREN, MIT MINDESTENS EINEM EPITOP EINER T-HELFERZELLE, UND IHRE ANWENDUNGEN ALS IMPFSTOFF**

**LIPOPEPTIDES INDUCING T LYMPHOCYTIC CYTOTOXICITY BEARING AT LEAST ONE AUXILIARY T EPITOPE, AND USES FOR VACCINATION**

(84) Etats contractants désignés:
**AT BE CH CY DE DK ES FI FR GB GR IE IT LI LU MC NL PT SE**

(30) Priorité: **07.04.1998 FR 9804323**

(43) Date de publication de la demande:
**17.01.2001 Bulletin 2001/03**

(73) Titulaires:
• **INSTITUT NATIONAL DE LA SANTE ET DE LA RECHERCHE MEDICALE (INSERM)**
**75654 Paris Cédex 13 (FR)**
• **CENTRE NATIONAL DE LA RECHERCHE SCIENTIFIQUE**
**75016 Paris Cédex (FR)**
• **INSTITUT PASTEUR DE LILLE**
**59019 Lille Cédex (FR)**

(72) Inventeurs:
• **LE GAL, Frédérique, Anne**
**F-94300 Vincennes (FR)**
• **GUILLET, Jean, Gérard**
**F-92170 Vanves (FR)**
• **GAHERY-SEGARD, Hanne**
**F-75014 Paris (FR)**
• **GRAS-MASSE, Hélène**
**F-59710 Merignies (FR)**

• **MELNYK, Oleg**
**F-59370 Mons-en-Baroeul (FR)**
• **TARTAR, André**
**F-62490 Vitry-en-Artois (FR)**

(74) Mandataire: **Catherine, Alain et al**
**Cabinet Harlé & Phélip**
**7, rue de Madrid**
**75008 Paris (FR)**

(56) Documents cités:
**EP-A- 0 433 242          EP-A- 0 491 628**
**WO-A-95/22317          GB-A- 2 271 995**
**US-A- 5 637 481**

• **VITIELLO A ET AL: "Development of a lipopeptide -based therapeutic vaccine to treat chronic HBV infection. I. Induction of a primary cytotoxic T lymphocyte response in humans" JOURNAL OF CLINICAL INVESTIGATION, vol. 95, no. 1, janvier 1995, pages 341-349, XP002075038 cité dans la demande**
• **J.P. SAUZET ET AL: "Long lasting anti-viral cytotoxic T lymphocytes induced in vivo with chimeric-multirestricted lipopeptides" VACCINE, vol. 13, no. 14, 1995, pages 1339-1345, XP002089324**

## Description

La présente invention concerne des lipopeptides inducteurs de cytotoxicité T lymphocytaire et comprenant au moins un épitope T auxiliaire. Elle est en outre relative à l'utilisation de ces lipopeptides comme vaccin.

Il existe deux types de réponses immunitaires: la réponse humorale due aux anticorps, et la réponse cytotoxique due aux lymphocytes T CD8[+].

Une réponse cytotoxique efficace requiert la présentation des antigènes aux lymphocytes T cytotoxiques CD8[+] (CTL), en association avec les molécules de classe I du Complexe Majeur d'Histocompatibilité (MHC), mais aussi aux lymphocytes T auxiliaires CD4[+] (HTL) en association avec les molécules de classe II du MHC.

L'utilisation de lipopeptides pour l'induction d'une réponse cytotoxique, c'est-à-dire la génération in vivo de lymphocytes T cytotoxiques a déjà été décrite: En particulier, la demande FR-90 15 870 (publication 2.670 787.) (Institut Pasteur de Lille, Institut Pasteur, INSERM) décrit des lipopeptides constitués d'une partie peptidique comprenant de 10 à 40 acides aminés et d'une partie lipidique qui peut être dérivée d'acides gras ou de groupements stéroïdes.

Ces lipopeptides montrent une bonne aptitude à induire une réponse cytotoxique. Il convenait cependant de les rendre capables d'induire également une réponse T auxiliaire, dont on sait l'importance pour l'induction et le maintien de la réponse cytotoxique.

A la connaissance des demandeurs, seul un article au nom de **VITIELLO et al.** (1995, J. Clin. Invest.,95, 341-349) a évoqué la possibilité de combiner sur une même molécule lipopeptidique un épitope CTL et un épitope induisant une réponse auxiliaire (épitope T-HELPER ou HTL).

La synthèse de deux lipopeptides est décrite dans cet article. Le premier est constitué de la partie 18-27 du core du virus de l'hépatite B, en tant qu'épitope CTL, de la partie 830-843 de la toxine tétanique, comme épitope T auxiliaire, et de deux chaînes palmitoyles. Les auteurs observent l'induction d'une cytotoxicité T-lymphocytaire.

Le second lipopeptide est constitué de l'épitope NP 147-155 du virus influenza murin, d'un épitope T auxiliaire et de chaînes lipidiques.

Néanmoins, les lipopeptides décrits dans cet article présentent une faible solubilité, due à la présence d'une part de la partie lipidique, et d'autre part de motifs peptidiques hydrophobes. On notera à ce sujet que les produits décrits par ces auteurs sont stockés en solutions mères dans du DMSO concentré, et dilués extemporanément pour l'injection dans une solution aqueuse tamponnée.

Cette forte hydrophobicité rend difficile la fabrication de solutions de ces lipopeptides, et de ce fait limite considérablement les possibilités de stérilisation par filtration, qui est la méthode généralement utilisée en raison de sa facilité de mise en oeuvre. Elle limite en outre fortement toute possibilité de caractérisation.

Le problème à résoudre consistait donc à réduire l'hydrophobicité des lipopeptides multi-épitopes tout en maintenant l'accessibilité à l'apprêtement nécessaire à l'acheminement du motif épitope CTL vers le CMH de classe I.

La présente invention a pour objet de résoudre ce problème.

Les inventeurs ont maintenant trouvé qu'il était possible non seulement d'augmenter l'hydrophilie des molécules lipopeptidiques, mais aussi de limiter des interactions entre les différents épitopes, en introduisant des séquences hydrophiles d'acides aminés, appelées espaceurs, au sein de ces molécules.

La présente invention a donc pour objet des lipopeptides comprenant au moins un épitope T auxiliaire, au moins un épitope CTL et au moins un résidu lipidique caractérisés en ce que les épitopes et la partie lipidique d'une part, et les épitopes d'autre part, sont séparés indépendamment par des séquences d'acides aminés, appelées espaceurs, au moins l'un des espaceurs comprenant des enchaînements de 1 à 10 acides aminés choisis parmi les acides aminés glycine, arginine, acide glutamique et acide aspartique. Ces enchaînements d'acides aminés globalement chargés en milieu neutre, assurent l'hydrophilie du lipopeptide.

De manière préférentielle, les espaceurs sont accessibles à un apprêtement protéolytique par le protéasome, préalable à la libération du motif épitope CTL. Cette accessibilité peut être mise en évidence comme décrit par **OSSENDORP et al.** (1996, Immunity, 5, 115-124).

De manière préférentielle les espaceurs comprennent entre 2 et 4 acides aminés, le nombre d'acides aminés étant suffisant pour obtenir des espaceurs globalement chargés en milieu neutre. Préférentiellement, au moins un des espaceurs comprend des arginines et/ou des glycines. Les arginines présentent l'avantage d'être chargées aux pH physiologiques, et de créer des sites de sensibilité particulière au clivage protéolytique lors de l'apprêtement des épitopes.

Les glycines présentent l'avantage de permettre l'introduction, au sein de la partie peptidique du lipopeptide, d'un site éventuel pour une synthèse convergente par couplage de fragment.

Selon un mode de mise en oeuvre particulièrement avantageux de l'invention, au moins un des espaceurs présente l'une des séquences suivantes:

## GLY ARG ou ARG GLY ARG.

L'acide glutamique et/ou l'acide aspartique présentent également d'intéressantes propriétés applicables à la réalisation d'espaceurs selon la présente invention (accès à la dégradation protéolytique naturelle, et introduction de fonctions carboxylates ionisées en milieu physiologique à pH neutre).

Ces acides aminés, rentrant dans la séquence des espaceurs, peuvent avantageusement être remplacés par leurs dérivés, ou par d'autres acides aminés fonctionnellement équivalents.

Ces acides aminés peuvent, au sein des espaceurs, être séparés par des acides aminés peu encombrés d'un point de vue chimique, c'est-à-dire présentant des chaînes latérales courtes, qui peuvent être, outre la glycine, l'alanine. La présence de ces acides aminés à chaîne courte facilite la protéolyse.

Une cystéine, ou une chaîne alkyle fonctionnalisée par un groupe thiol, capable de ménager un site possible de ligation chimique simple entre deux fragments peptidiques grâce à la formation de liaisons covalentes non peptidiques peut aussi être introduite dans l'espaceur. Il est ainsi possible de réaliser une liaison disulfure entre deux peptides différents, comportant chacun une fonction thiol, ou une liaison thioéther, auquel cas l'un des deux peptides doit être fonctionnalisé par un halogénure d'alkyle.

Les deux peptides peuvent aussi être liés par formation d'un hétérocycle thiazolidine l'un des peptides amenant une fonction aldéhyde. Cette fonction aldéhyde peut être facilement et sélectivement générée sous forme d'un groupement alpha-oxo acyle, obtenu par oxydation périodique d'une sérine, d'une thréonine, ou d'une cysteine introduite en position N-terminale d'un fragment peptidique

Il est aussi possible d'associer les deux peptides en utilisant la réactivité des aldéhydes avec des bases faibles, ou des méthodes de ligation via la formation d'une oxime (par réaction entre une fonction aldéhyde et une fonction amino-oxyacétyle), d'une hydrazone (par réaction entre une fonction aldéhyde et une hydrazide ou une aryl-hydrazine). Ces réactions de base sont rappelées dans la revue de James Tam et Jane Spetzler.(Biomed. Pep., Prot. and Nucleic Acids (1995), 1, 123-132).

Une méthode de ligation consistant à générer une liaison hydrazone à partir d'une alkyl-hydrazine (générée par N-amination d'un précurseur amine) et d'un partenaire fonctionnalisé par un groupement alpha-oxo acyle a été récemment développée.

Les hydrazinopeptides sont synthétisés par N-amination selon Klinguer et al. (Tet. Lett. (1996), 37, 7259-7262). Cette méthodologie permet de transformer n'importe quelle fonction amine du peptide en fonction hydrazine. La fonction hydrazine peut ainsi être située en position N-terminale, ou sur la chaîne latérale d'un acide aminé situé à n'importe quel endroit de la séquence. La fonction amine transformée peut être une fonction amine alpha, epsilon d'une lysine ou de l'acide aminocaproïque, une fonction delta amino d'une omithine, ou toute autre fonction amine primaire ou secondaire.

Les peptides aldéhydes sont générés comme décrits par Tam et Spetzler ( Biomed. Pep., Prot. and Nucleic Acids (1995), 1, 123-132). Les résidus sérine, thréonine ou cystéine précurseurs du groupement alpha-oxo acyle peuvent être situés en position N-terminale, ou sur toute fonction amine d'une chaîne latérale à partir du moment ou ces résidus ne sont pas présents en position N-terminale dans les épitopes considérés.

Le groupement lipidique peut être porté indifféremment par l'hydrazinopeptide ou le peptide aldéhyde.

On entend, pour la compréhension de la présente invention, par épitope T auxiliaire, une séquence d'acides aminés capable de s'associer à au moins un récepteur HLA de classe II.

On entend par épitope CTL une séquence d'acides aminés capable de s'associer à au moins un récepteur HLA de classe I.

Les épitopes T auxiliaires capables de s'associer à plusieurs récepteurs HLA de classe II différents sont appelés épitopes auxiliaires multivalents (HTL multivalents).

Les lipopeptides selon la présente invention comprennent préférentiellement l'enchaînement de:

- une partie lipidique;
- un premier espaceur;
- un épitope T auxiliaire;
- un second espaceur; et
- un épitope CTL.

Ils peuvent aussi comprendre :

- une partie lipidique;
- un premier espaceur;

- un épitope T auxiliaire;
- un second espaceur;
- un épitope CTL;
- un troisième espaceur; et
- un second épitope CTL.

Les divers épitopes auxiliaires et CTL peuvent être présents dans des ordres différents dans l'enchaînement d'acides aminés. Ainsi, un épitope auxiliaire peut être compris entre deux épitopes CTL, desquels il sera séparé par deux espaceurs.

La partie lipidique du lipopeptide est avantageusement obtenue par addition d'un motif lipidique sur une fonction alpha aminée d'un peptide ou sur une fonction réactive de la chaîne latérale d'un acide aminé de la partie peptidique , telle qu'une fonction epsilon amine ou thiol. Elle peut comprendre une ou plusieurs chaînes dérivées d'acides gras en $C_{10}$ à $C_{20}$, éventuellement ramifiées ou insaturées ou un dérivé d'un stéroïde.

De manière avantageuse, la partie lipidique comprend au moins deux chaînes dérivées d'acides gras ou d'alcools gras en $C_{10}$ à $C_{20}$ liées aussi entre elles par l'intermédiaire d'un ou plusieurs acides aminés. La partie lipidique peut ainsi être constituée de deux chaînes d'acide palmitique liées aux groupements $NH_2$, alpha et epsilon, d'une lysine.

La partie lipidique peut aussi être constituée de, ou comprendre, un résidu d'acide palmitique, d'acide oléique, d'acide linoléique, d'acide linolénique, d'acide 2-amino hexadécanoïque, de pimélautide ou de triméxautide.

La partie non lipidique comprend quant à elle entre 15 et 100, et préférentiellement entre 15 et 50 acides aminés. Le nombre d'acides aminés dépend du nombre d'épitopes constituant la partie non lipidique du lipopeptide, et de leurs tailles.

De manière avantageuse, l'épitope T auxiliaire est un épitope capable de s'associer à plusieurs récepteurs HLA de classe II différents, c'est-à-dire un épitope multivalent. Il est préférentiellement l'épitope auxiliaire constitué par le peptide 830-843 de la toxine tétanique présentant la séquence suivante:

## QYIKANSKFIGITE

La glutamine (Q) de cette séquence peut éventuellement être acétylée.

D'autres épitopes HTL multivalents peuvent être l'épitope multivalent de l'hémagglutinine (**PREVOST-BLONDEL et al.** 1995, J. Virol., vol.62, n°12, pages 8046-8055) ou encore l'épitope PADRE (**ALEXANDER et al.,** 1994, Immunity, 1, 751).

L'épitope CTL peut quant à lui être tout épitope capable d'activer des lymphocytes T cytotoxiques CD8$^+$.

Il est préférentiellement un épitope CTL d'une protéine présentée par une cellule tumorale et en particulier par un mélanome, d'une protéine du VIH, du virus de l'hépatite B (VHB) ou du papillomavirus, ou encore de la protéine p53.

Il peut être en particulier l'un des épitopes suivants:

- épitopes de la protéine BCR-ABL, résultant de la translocation BCR- Abelson (leucémie myéloïde chronique) tels que mentionnés dans le tableau 1.
- épitopes de la protéine p53, tels que ceux mentionnés dans le tableau 2.

Les épitopes de la protéine p53 peuvent en outre être pris dans les séquences 25-35, 63-73, 129-156, 149-156, 187-205, 187-234, 226-264, ou 249-264 de cette protéine.

- épitopes des protéines $E_6$ ou $E_7$ du papillomavirus humain (VPH), tels que ceux mentionnés dans le tableau 3.
- épitopes de protéines du virus VIH-1 tels que ceux mentionnés dans le tableau 4,
- épitopes du mélanome ou d'autres tumeurs, tels que ceux mentionnés dans les tableaux 5, 6 et 7 et en particulier épitopes de l'antigène metan-A/mart-1 du mélanome.

D'autres épitopes CTL plurivalents présentant une capacité d'association avec des HLA de classe I peuvent être ceux compris dans le peptide 43-57 de HPV (GQAEPDRAHNIVTF) qui contient des épitopes HLA A2, A24, B7 et B18.

Les épitopes CTL peuvent encore être ceux d'antigènes parasitaires, et en particulier ceux d'une protéine du stade intra-hépatocytaire de *Plasmodium falciparum.*

La liaison entre la partie lipidique et la partie non lipidique est préférentiellement effectuée par l'intermédiaire du groupement $\varepsilon\text{-}NH_2$ du premier acide aminé de la partie non lipidique. Elle peut néanmoins être effectuée par tout autre moyen , et en particulier par le groupement $\alpha\text{-}NH_2$ d'une lysine.

Les lipopeptides selon la présente invention peuvent être administrés aux patients à traiter, en particulier aux personnes à vacciner, dilués dans un solvant adéquat, tel que par exemple un tampon physiologiquement acceptable. Ils peuvent néanmoins être mis sous une forme galénique compatible avec une administration par voie parentérale, sublinguale, intrapulmonaire ou transdermique.

Ils peuvent être administrés par tout mode d'administration permettant une action efficace. Ce mode sera choisi en fonction de la maladie à traiter. A titre non limitatif, les lipopeptides peuvent en particulier être administrés par injection ou par voie sublinguale.

Un autre objet de la présente invention est donc l'utilisation de ces lipopeptides pour la fabrication d'un médicament ou d'un vaccin, préventif ou curatif, pour l'induction d'une réponse immunitaire spécifique, et en particulier, pour l'induction d'une réponse immunitaire à l'encontre de cancers tels que le mélanome, des virus VIH et VHB, des papillomavirus, de la p53 ou de la malaria.

La présente invention a encore pour objet une composition pharmaceutique caractérisée en ce qu'elle contient une quantité pharmacologiquement active d'un ou plusieurs des lipopeptides décrits ci-dessus, ainsi que des excipients pharmaceutiquement compatibles.

On notera enfin que les épitopes CTL décrits dans la présente demande constituent en eux-mêmes des objets de la présente invention.

La présente invention est illustrée sans pour autant être limitée par les exemples qui suivent.

La figure 1 illustre la reconnaissance des lipopeptides monopalmitoyle et dipalmitoyle selon l'invention, comprenant l'épitope Mart 27-35, par une lignée de lymphocytes T cytotoxiques spécifiques dudit épitope dans un test Elispot-interféron gamma (IFN-γ).

Les figures 2A et 2B illustrent la reconnaissance des lipopeptides mentionnés pour la figure 1, par deux clones de lymphocytes infiltrants de métanomes, respectivement LT8 et LT12, spécifiques de l'épitope Mart 27-35.

La figure 3 illustre l'induction d'une activité T-auxiliaire par le peptide TT 830-843 constitutif des lipopeptides selon l'invention.

**-Figure 3a:** sécrétion d'Interféron-γ par des cellules de ganglions lymphatiques de souris transgéniques HLA-A2 auxquelles on a injecté:

(A) Tampon NaCl
(B) lipopeptide Mp-MART;
(C) Lipopeptide Dp-MART.

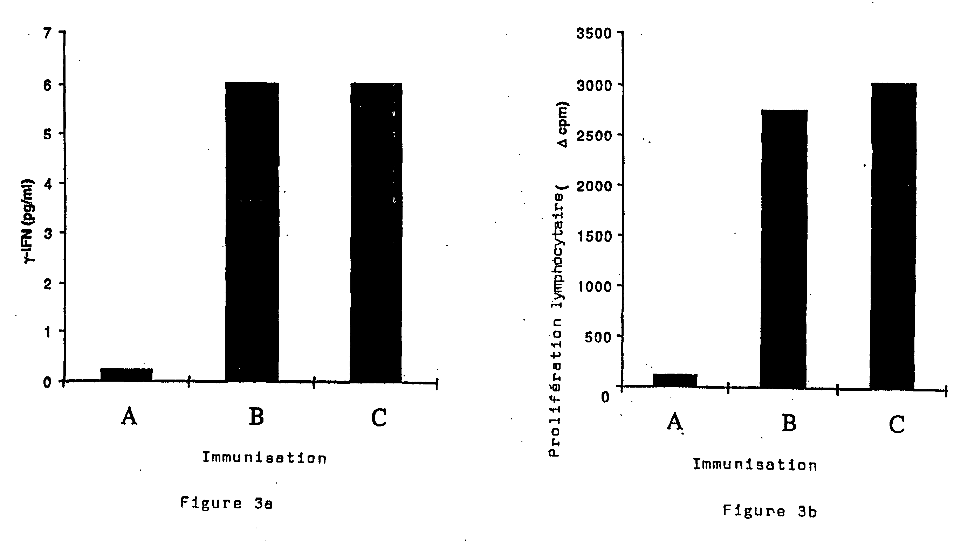

**[0059]** - **Figure 3b:** prolifération des cellules des ganglions lymphatiques de souris transgéniques HLA-A2 en réponse aux mêmes antigènes.

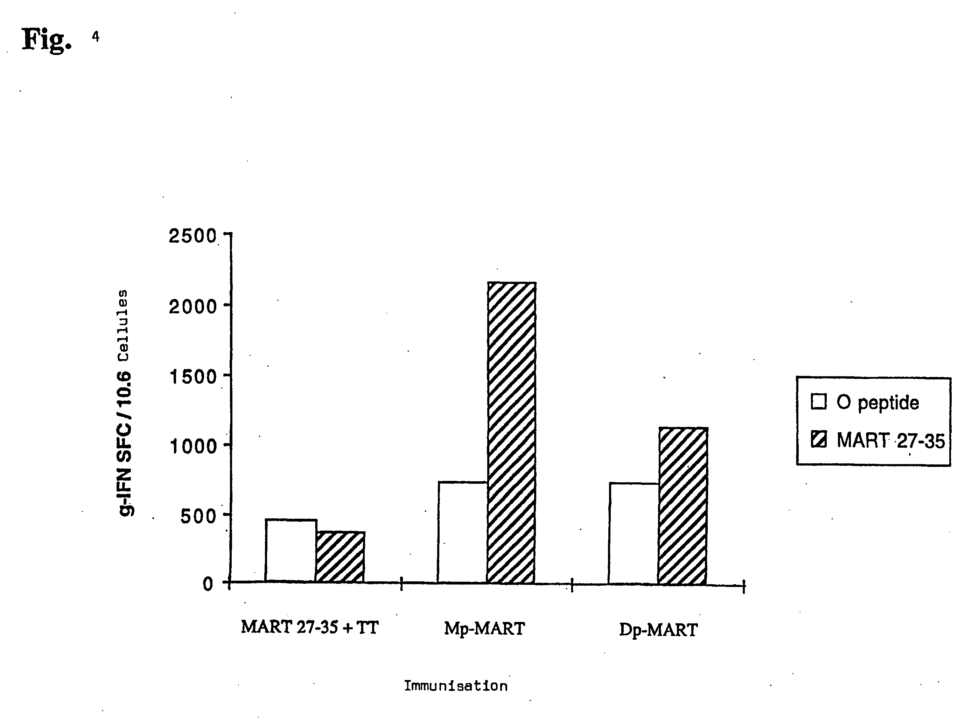

La figure 4 illustre l'induction de la sécrétion d'Interféron-γ par des cellules des ganglions lymphatiques de souris transgéniques HLA-A2 en réponse à:

(a) mélange des peptides MART 27-35 et TT 830-845;
(b) lipopeptide Mp-MART;
(c) lipopeptide Dp-MART.

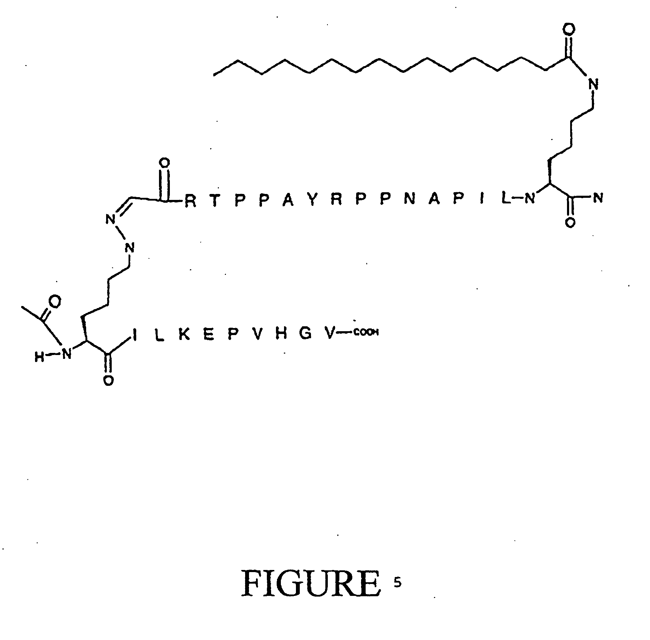

La figure 5 représente la formule d'un lipopeptide comprenant une liaison hydrazone.

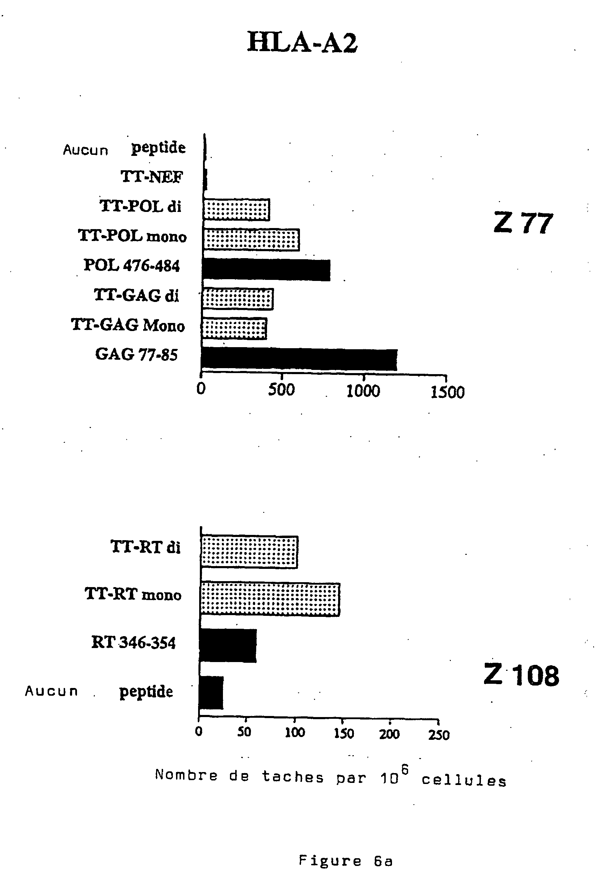

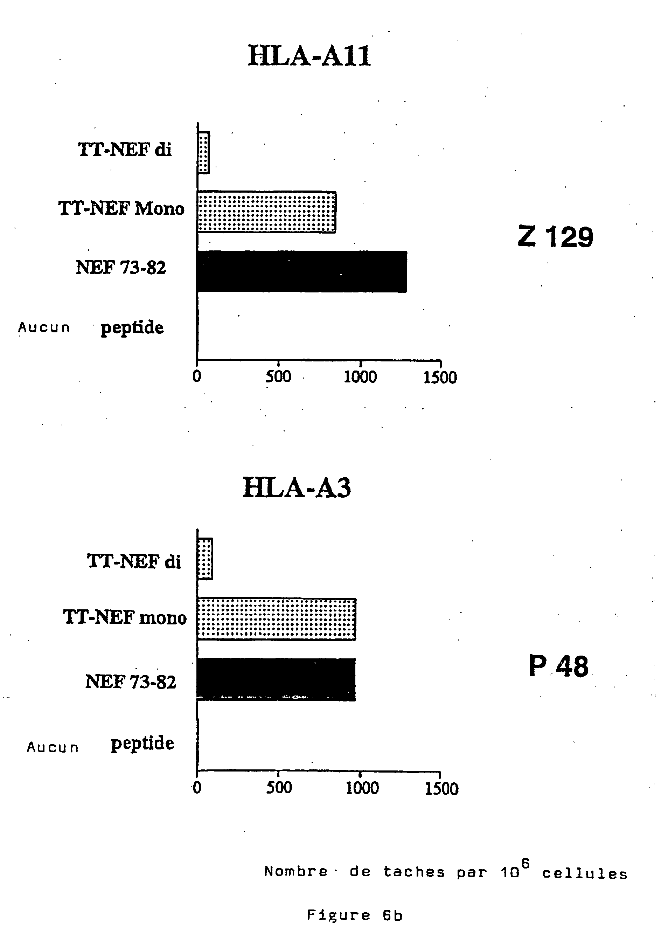

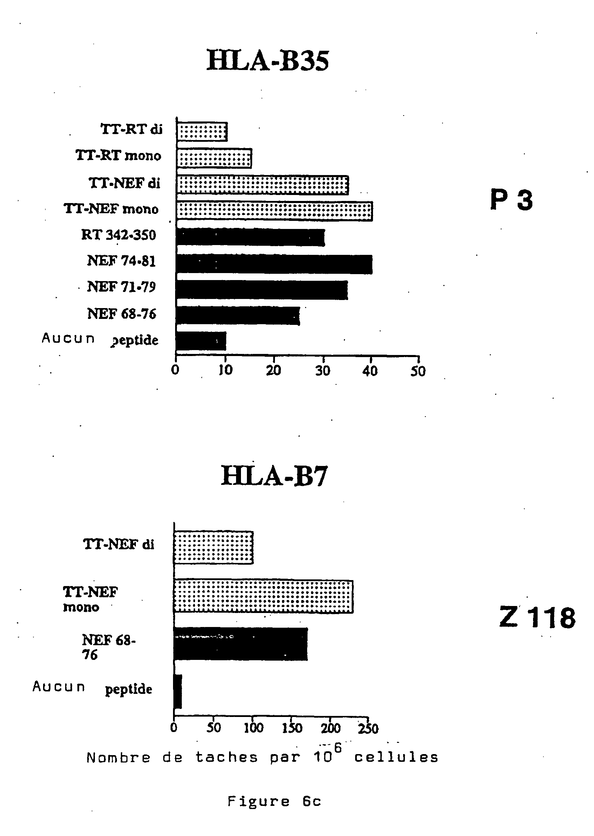

La figure 6 illustre l'antigénicité des épitopes HIV et de leurs lipopeptides correspondants.

Des PBMC de patients séropositifs pour HIV ont été incubés avec les peptides sélectionnés (barres pleines) ou les lipopeptides correspondants (barres pointillées). L'activation des CTL spécifiques a été suivie en utilisant le test ELISPOT spécifique de l'Interféron-γ. Les codes affectés à chaque patient et leur type HLA sont indiqués. Les données sont représentatives de deux expériences indépendantes.

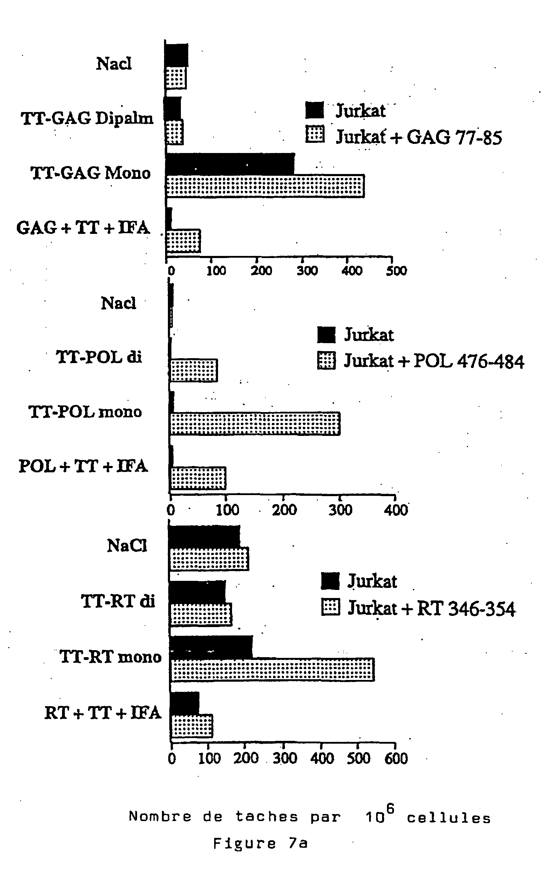

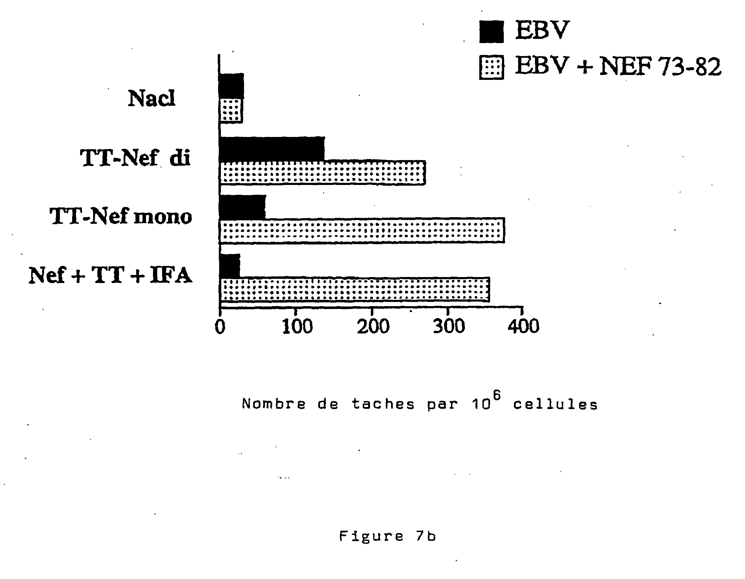

La figure 7 illustre l'immugénicité des épitopes restreints au HLA-A2 et HLA-A3 et les lipopeptildes correspondants.

(a) les peptides ou lipopeptides indiqués ont été injectés à des souris transgéniques HLA-A2 . Avant injection à la base de la queue, les peptides ont été mélangés avec l'épitope T-auxiliaire TT 830-843 et émulsifiés dans de l'adjuvant incomplet de Freund; simultanément les lipopeptides ont été dilués dans du tampon physiologique. La production de CTL spécifique a été déterminée à l'aide du test ELISPOT spécifique de l'interféron-γ, après incubation des cellules des ganglions lymphatiques avec des cellules cibles de type Jurkat-A2 $K^B$ préincubés en présence (barre pointillée) ou en l'absence (barre pleine) de l'épitope CTL indiqué.

(b) les peptides ou les lipopeptiques indiqués ont été injectés à la base de la queue de souris transgéniques HLA-A3 . La production de CTL spécifiques a été déterminée par le test ELISPOT spécifique de l'Interféron-γ, après incubation des cellules des ganglions lymphatiques avec des cellules cibles EBV exprimant le HLA-A3 préincubé en présence (barre pointillée) ou en l'absence (barre pleine) de l'épitope NEF 73-81 de HIV.

Les résultats présentés sont représentatifs de trois expériences indépendantes.

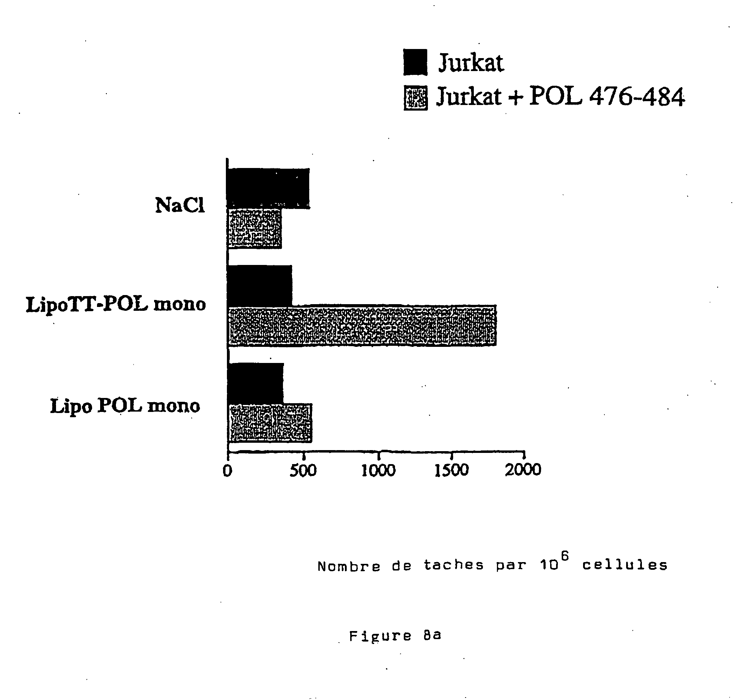

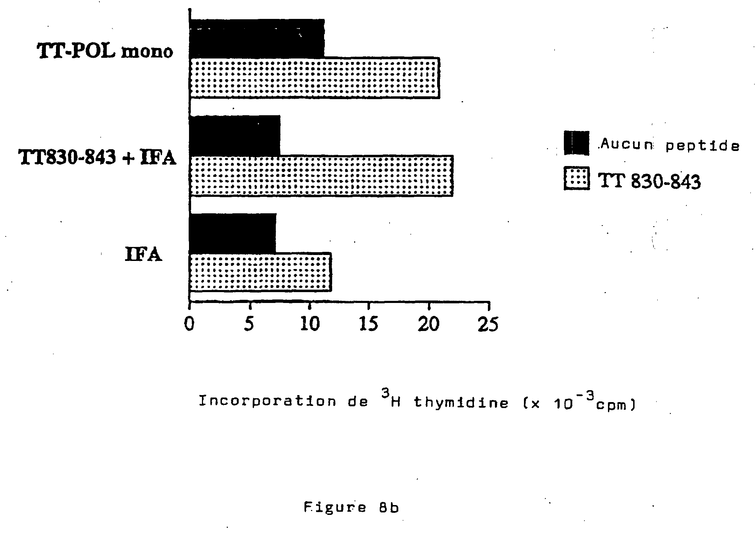

La figure 8 illustre la nécessité d'un épitope T-auxiliaire de type TT 830-843 pour l'induction de CTL.

(a) Des lipopeptides monopalmitoyle comprenant l'épitope CTL POL 476-484 de HIV dépourvus (lipo POL1) ou non (lipo TT-POL1) de l'épitope T-auxiliaire TT 830-843 ont été dilués dans un tampon physiologique et injectés à la base de la queue de souris transgéniques HLA-A2. Les résultats sont représentatifs de deux expériences indépendantes.

(b) Des cellules des ganglions lymphatiques de souris immunisées avec le lipopeptide monopalmitoyle TT-POL1, ou le lipopeptide monopalmitoyle TT 830-843 émulsifié dans de l'adjuvant incomplet de Freund, ou injecté avec de l'adjuvant incomplet de Freund seul, ont été co-cultivées pendant 72 heures en présence (barre pointillée) ou en l'absence (barre pleine) 30µg/ml de l'épitope T-auxiliaire TT 830-843.

La prolifération cellulaire a été suivie par la mesure de l'incorporation de $^3$H thymidine . Les résultats sont exprimés comme la moyenne de la radio-activité (cpm) des cultures (3 puits par point) d'au moins trois expériences.

**Exemple 1 Synthèse de lipopeptides dipalmitoylés avec et sans espaceur, et propriétés physico-chimiques**

**1. Synthèses des lipopeptides**

La série des trois lipopeptides suivants a été synthétisée.

## Lipopeptide N° 1

## Pam-K(Pam)-SS-QYIKANSKFIGITE-AAA-AAGIGILTV

## Lipopeptide n°2

## Pam-K(Pam)-SS-QYIKANSKFIGITE-RGR-AAGIGILTV

## Lipopeptide n°3

## Pam-K(Pam)-GR-QYIKANSKFIGITE-RGR-AAGIGILTV

Ces lipopeptides comprennent une extrémité lipidique constituée de deux résidus palmitoyl (Pam) liés au groupement $NH_2$ d'une lysine, l'épitope auxiliaire de la toxine tétanique (peptide 830-843) et un épitope CTL reconnu dans mart-1 par le récepteur HLA A2.1. L'épitope Hbc 18-27 décrit par VITIELLO et al. (cité supra) est aussi reconnu par HLA A2.1, et présente une hydrophobicité similaire au motif choisi dans la série de lipopeptides précitée.

La différence réside dans les séquences d'acides aminés comprises d'une part entre la partie lipidique et l'épitope auxiliaire et d'autre part, entre l'épitope auxiliaire et l'épitope CTL. Cette série a été réalisée pour évaluer la possibilité d'obtenir sous forme purifiée et caractérisable des produits définis par référence au produit selon **VITIELLO** et comportant comme celui-ci deux chaînes palmitoyles: les deux espaceurs de **VITIELLO** sont reproduits ou modifiés pour l'un ou les deux espaceurs selon la présente invention.

Dans le lipopeptide n° 1, ces séquences sont identiques à celles du lipopeptide décrit par **VITIELLO et al..** Deux sérines sont insérées entre la partie lipidique et l'épitope auxiliaire. Trois alanines sont quant à elles placées entre l'épitope auxiliaire et l'épitope CTL.

Le lipopeptide n°2 comprend les séquences SS ( séryl-séryl) et **ARG - GLY - ARG.**

Le lipopeptide n° 3 est construit selon la présente invention, et comprend des espaceurs hydrophiles présentant respectivement les séquences **GLY-ARG et ARG-GLY-ARG.**

La synthèse a été réalisée selon des procédures « standards » utilisées en phase solide selon la stratégie Boc-benzyle décrite par **Merrifield** ( 1963 J. Am. Chem. Soc. 85, 2149-2154; 1986 Science 232, 341-347). L'introduction en position N-terminale d'une di-Boc Lysine permet, après déprotection de la peptidyl résine, l'introduction simultanée des deux chaînes palmitoyle, pour obtenir le dérivé attendu.

**2. Purification et propriétés physico-chimiques des lipopeptides.**

Le lipopeptide n° 1 est pratiquement insoluble dans l'eau, ou dans un mélange DMSO/eau 10% V/V. Il s'avère non purifiable, non caractérisable par HPLC selon des méthodes résolutives, en raison de la formation d'agrégats. L'identité de ce lipopeptide a pu être confirmée par mesure de la masse par spectrométrie PDMS-TOF, réalisée sur le produit brut, immédiatement après le clivage terminal par l'acide fluorhydrique et avant lyophilisation. Le produit devient totalement insoluble dans l'eau après lyophilisation.

La mesure de la masse par spectrométrie PDMS-TOF du lipopeptide n°2 est possible, et confirme que le produit attendu a bien été obtenu. Cependant, ce produit adopte dans l'eau un comportement voisin du lipopeptide n°1, et ne peut donc être correctement analysé.

Le lipopeptide n° 3 est soluble dans l'acide acétique à 80%. Il peut être stérilisable par filtration sur un microfiltre de 0,22 µm. Il peut être en outre dissous dans une solution DMSO/eau 10% V/V. La mesure de la masse par spectrométrie PDMS-TOF confirme la présence du produit attendu. Le produit s'avère purifiable après injection d'une solution concentrée dans le DMSO, sur colonne Vydac C4, à 60°C, en utilisant un gradient d'acétonitrile, en présence du contre-ion TFA. Le produit est caractérisable par HPLC selon plusieurs méthodes résolutives conventionnelles, sur colonnes Vydac C4, Zorbax C3, Zorbax CN, Zorbax C1, à 60°C, à l'aide de gradients d'acétonitrile en présence d'un contre-ion TFA, ou en utilisant un modificateur organique, tel que l'isopropanol ou le butanol en isocratique.

Le trifluoroacétate du lipopeptide n°3 est soluble dans le DMSO (20-25 mg/mL), et reste soluble après dilution par de l'eau (DMSO 10%). L'échange du contre-ion trifluoroacétate pour un contre-ion acétate peut être réalisé par RP-HPLC sur une colonne C4: le produit dissous dans le DMSO à 80% est injecté sur la colonne équilibrée par le solvant A (acide acétique à 5% dans l'eau). Après élimination des produits non retenus (contre-ions TFA, sels), le produit est élué par l'aide d'un gradient du solvant A vers le solvant B (acétonitrile 80% - acide acétique à 5% - eau 15%).

L'acétate présente une solubilité comparable à celle du trifluoroacétate. Il a été utilisé pour immuniser des souris transgéniques exprimant HLA-A2, et s'est avéré capable d'induire une réponse CTL satisfaisante en l'absence d'adjuvant d'immunisation.

Ces résultats montrent donc que l'insertion d'espaceur hydrophile entre la partie lipidique et l'épitope auxiliaire d'une part, et d'autre part entre l'épitope auxiliaire et l'épitope CTL permet de solubiliser le lipopeptide, ce qui n'est pas le cas quand les séquences d'acides aminés sont différentes.

**EXEMPLE 2: Synthèse de lipopeptides monopalmitoylés selon l'invention, et propriétés physico-chimiques:**

Une deuxième série de lipopeptides a été réalisée pour évaluer la possibilité d'obtenir sous forme purifiée et caractérisable des produits comportant cette fois-ci une seule chaîne palmitoyle; l'espaceur central employé est introduit selon la présente invention (-RGR- pour arginyl-glycyl-arginyl-), tandis que l'espaceur -SS-(seryl-seryl-) de **VITIELLO** est maintenu ou remplacé pour l'un ou les deux espaceurs selon la présente invention.

L'introduction en position N-terminale d'une alpha-Fmoc, epsilon Boc Lysine permet, après déprotection sélective du groupe Boc, l'introduction d'une seule chaîne palmitoyle, pour obtenir le produit suivant:

## Lipopeptide n°4:

## H-K(Pam)-SS-QYIKANSKFIGITE-RGR-AAGIGILTV

Ce produit s'avère difficilement purifiable. Il n'est soluble en milieu aqueux qu'en présence de DMSO. Un profil chromatographique peut être obtenu par RP-HPLC uniquement sur une colonne de type C1 en utilisant un système solvant « standard » (acétonitrile/eau/acide trifluoroacétique), selon une méthode peu résolutive, qui ne permet pas de garantir la réelle pureté du produit.

Le lipopeptide n°5 suivant, a été synthétisé.

## Lipopeptide n°5:

## H-K(Pam)-GR-QYIKANSKFIGITE-RGR- AAGIGILTV

Ce produit a été obtenu comme le lipopeptide n°4 par acylation sélective de la fonction epsilon $NH_2$ de la lysine N-terminale.

Ce lipopeptide n°5 satisfait au critère de purification et de caractérisation classiques.

Cette comparaison entre les lipopeptides n°4 et n°5, qui sont des lipopeptides monopalmitoyles, confirme les résultats obtenus sur les lipopeptides dipalmitoyles.

Les lipopeptides 3 et 5 ont été les seuls à répondre aux critères définis (accès à une méthode de purification et à des critères analytiques résolutifs). Leur étude a été poursuivie par l'étude de leur reconnaissance par divers types cellulaires. Les résultats de cette étude figurent dans l'exemple 3.

**Exemple 3. Activité biologique de lipopeptides dipalmitoyle et monopalmitoyle selon l'invention contenant un épitope CTL mélanome**

Le lipopeptide dipalmitoyle n°3 et le lipopeptide monopalmitoyle n°5 synthétisés comme indiqué respectivement dans les exemples 1 et 2 ont été testés.

**1°) Etude de la reconnaissance des lipopeptides mono- et dipalm-Mart 27-35 par une lignée de lymphocytes T cytotoxiques humains spécifiques de MART 27-35 dans un test Elispot Interféron-$\gamma$ (IFN-$\gamma$).**

**a) Matériels et méthodes:**

La lignée L28.3 de lymphocytes T cytotoxiques spécifiques de MART 27-35 a été obtenue à partir de cellules mononuclées du sang périphérique (PBMC) de donneur sain HLA-A2$^+$. Le protocole d'induction de ces cellules effectrices à partir de PBMC de donneur naïf a été décrit par **Ostankovitch et al.** (Int. J. Cancer, 1997, 72, 987-994).

**Méthode du test Elispot:**

Dans ce test les cellules cibles sont des PBMC autologues non stimulées (naïfs).

50 µl/puits d'anticorps murin anti-IFN$\gamma$ humain dilué dans du tampon PBS à une concentration de 4 µg/ml sont incubés dans des plaques de 96 puits à fond de nitrocellulose pendant la nuit à 4°C en chambre humide.

Les PBMC sont décongelées, maintenues au repos durant 1 heure dans du milieu RPMI contenant 10% de sérum humain AB (SAB) à 37°C dans une atmosphère, contenant 5% de $CO_2$. Elles sont ensuite incubées durant une nuit avec les peptides à tester à différentes concentrations (10, 5 et 1 µg/ml).

Les puits sont lavés avec du PBS puis saturés avec du milieu RPMI contenant 10% de SAB, pendant 2 heures à 37°C.

Les cellules effectrices sont ensuite distribuées dans les plaques de 96 puits préalablement incubées avec l'anti-IFN$\gamma$ à raison de 20.000 cellules par puits dans 200µl final et en présence des PBMC autologues stimulées avec un rapport effecteur: cible (E : T) de 1:1 . La phytohémagglutinine (PHA) à la concentration de 4 µg/ml et le phorbol 12-méristate 13-acétate (PMA)-ionomycine (150 et 500 ng/ml respectivement) sont utilisées à titre de témoins positifs.

Après 20 heures d'incubation à 37°C dans une atmosphère contenant 5% de $CO_2$, les puits sont lavés 5 fois avec du PBS puis 1 fois avec de l'eau distillée, puis sont incubés toute la nuit à 4°C avec 100µl/puits d'anticorps de lapin anti-IFN$\gamma$ humain dilué au 1/250ème dans du RPMI contenant 10% de SAB. Les puits sont ensuite lavés 5 fois avec du PBS contenant 0,05% de Tween 20, puis incubés pendant 2 heures à 37°C avec 100µl d'anticorps de chèvre anti-immunoglobuline de lapin biotinylés dilués au 1/500ème dans du PBS contenant 0,05% de Tween 20 et 1% de BSA (sérum albumine bovine).

Les puits sont à nouveau lavés cinq fois avec du PBS-Tween puis incubés pendant 1 heure à 37°C avec 100µl d'ExtrAvidine-Phosphatase Alcaline diluée au 1/600ème dans du PBS-Tween-BSA 1%.

Après quatre lavages en PBS-Tween, les puits sont incubés avec 100µl de substrat de révélation (Alkaline phosphatase conjugate substrate kit, Réf. 170-6432. Biorad). Les puits sont ensuite lavés avec de l'eau distillée et séchés avant la lecture des résultats au stéréomicroscope.

**b) Résultats:**

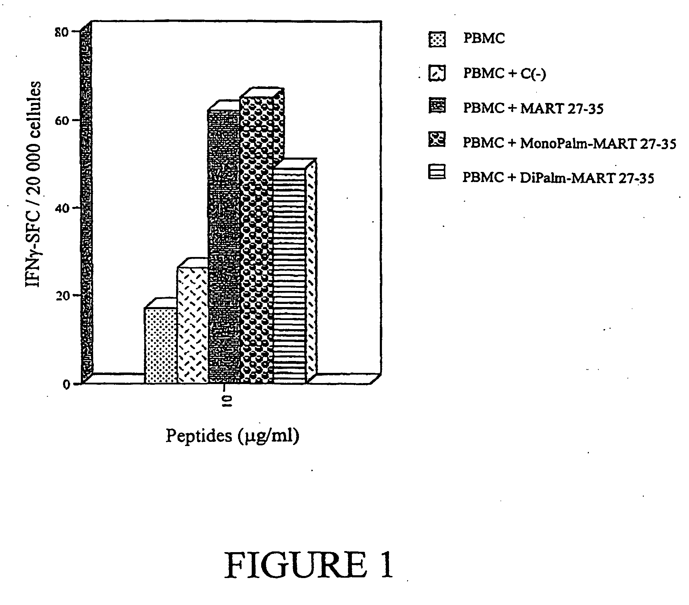

La figure 1 illustre les résultats obtenus.

Les deux lipopeptides de MART 27-35 stimulent de façon spécifique la sécrétion d'IFNγ par la lignée de CTLs anti-MART 27-35. Ils sont donc présentés par les PBMC et reconnus par les CTLs d'une façon comparable au peptide nominal MART 27-35.

**2) Etude de la reconaissance des lipopeptides mono- et dipalmitoyle MART 27-35 par deux clones de lymphocytes infiltrants de mélanomes humains spécifiques de MART 27-35, par un test de cytotoxicité par relarguage de $^{51}$Cr.**

**a) Matériels et méthodes:**

LT 8 et LT 12 sont deux clones HLA-A2$^+$ obtenus par la restimulation de lymphocytes infiltrants de mélanome (TILs), qui reconnaissent spécifiquement le peptide MART 27-35.

Les cellules cibles utilisées dans ce test sont des cellules humaines HLA-A2$^+$ de type T2. Ces cellules sont dépourvues de transporteurs de peptide et ne présentent donc que les peptides exogènes sur leurs molécules de classe I libres.

Dans ce test, les cellules cibles sont incubées durant une nuit avec le peptide à tester à raison de 4 μg/10$^6$ cellules, dans une atmosphère contenant 5% de $CO_2$ à 37°C. Elles sont ensuite incubées pendant une heure à 37°C avec 100 μCi de chromate de sodium ($^{51}$Cr). Les cellules cibles sont ensuite lavées deux fois dans du sérum physiologique contenant 5% de sérum foetal de veau (SVF), reprises dans du milieu RPMI 5% SVF et distribuées dans des plaques de 96 puits à raison de 3000 ou 5000 cellules par puits dans 100μl.

Les cellules effectrices (LT8 et LT12) ont ensuite été ajoutées dans 100μl du même milieu avec un rapport effecteur : cible de 10:1.

Le relarguage de chrome obtenu pendant l'incubation de 4 heures à 37 °C a été mesuré sur un compteur gamma. Le pourcentage de lyse est déterminé par la formule suivante (R = relarguage):

$$\% \text{ de lyse (R. expérimental - R. spontané/R. total- R.spontané) x 100.}$$

**b) Résultats:**

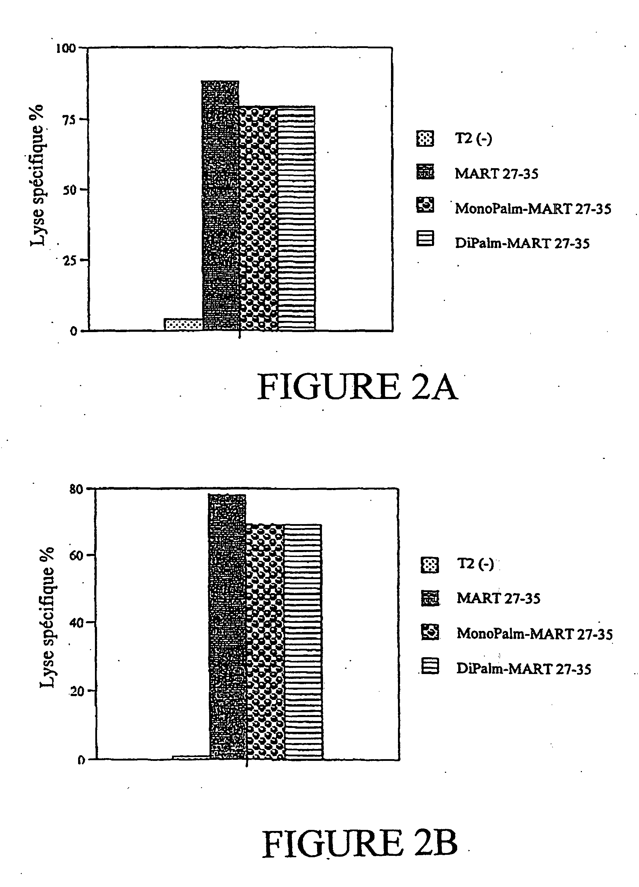

Les figures 2A et 2B illustrent les résultats obtenus.

Les deux lipopeptides de MART 27-35, mono et dipalmitique, sont reconnus par des cellules effectrices humaines, provenant de mélanomes et spécifiques du peptide MART 27-35. Ces deux types de lipopeptides sont reconnus de façon similaire, et à un niveau au moins comparable au peptide original (MART 27-35) compte-tenu que les cellules cibles ont été exposées à la même concentration finale de peptides alors que la masse molaire des lipopeptides est environ 5 fois plus élevée que celle du peptide MART 27-35.

**3. Caractérisation de l'effet « Helper » de l'épitope T auxiliaire TT 830-843.**

**a) Matériels et méthodes:**

Les souris transgéniques A2/Kb (3 à 4 par groupe) ont été immunisées en IFA par voie sous cutanée à la base de la queue et dans les coussinets plantaires avec soit (A) du sérum physiologique soit (B) 200 μg de Mp-MART, soit (C ) 200μg de Dp-MART. Les souris furent sacrifiées 11 jours plus tard.

Un test de prolifération des cellules des ganglions drainants a été réalisé par trois jours de culture en présence du peptide TT 830-843 (10 μg/ml) puis 18 heures d'incorporation de thymidine tritiée.

Le surnageant a été récolté après 36 heures de culture pour le dosage d'IFN-γ par méthode ELISA.

**b) Résultats:**

Afin de définir la capacité d'induction d'une réponse T auxiliaire apportée par les constructions de lipopeptides. selon l'invention, la sécrétion d'interféron-γ ainsi que la prolifération de lymphocytes en réponse a l'épitope T auxiliaire TT 830-843 a été étudiée chez des souris immunisées avec Mp-MART ou Dp-MART en présence d'adjuvant incomplet de Freund.

Les résultats sont reportés dans la figure 3.

Les résultats indiquent que la construction Mp-MART et la construction Dp-MART (B, C)induisent toutes les deux une forte sécrétion d'interféron-γ par les cellules des ganglions lymphatiques des souris transgéniques de type A2/K$^b$.

De même, les lipopeptides Mp-MART et Dp-MART (B, C) provoquent une prolifération lymphocytaire importante en réponse au peptide TT 830-843.

Ces résultats démontrent que l'épitope T auxiliaire TT 830-843 est capable d'induire une réponse T auxiliaire chez les souris transgéniques A2/K$^b$ . De plus, ces résultats montrent également que la liaison covalente de l'épitope TT830-843 au sein de la construction lipopeptidique ne réduit pas sa capacité à être présentée aux cellules T.

**4)Etude l'effet adjuvant de la partie lipidique des constructions de lipopeptides MART 27-35.**

**a) Matériels et méthodes:**

Les souris transgéniques A2/Kb (3 à 4 par groupe) ont été immunisées sans adjuvant par voie sous cutanée à la base de la queue et dans les coussinets plantaires avec soit (A) un mélange de peptide MART 27-35 (50 μg) et de TT 830-843 (140 μg), soit (B) 200 μg de Mp-MART (pour assurer l'équimolarité du peptide MART 27-35 par rapport à A), soit (C ) 200 μg de Dp-MART. Les souris furent sacrifiées 11 jours plus tard.

Le nombre de cellules T CD8$^+$ spécifiques de MART 27-35 a été déterminé par un test ELISPOT IFN-γ: au cours duquel les cellules ont été exposées à des cellules présentatrices de type Jurkat A2/Kb, soit non chargées à titre de contrôle négatif, soit chargées avec le peptide MART 27-35 (30 μg/ml). Les résultats sont exprimés en nombre de cellules sécrétant de l'IFN-γ par million de cellules testées.

**b) Résultats:**

Afin d'optimiser l'utilisation des constructions colinéaires de lipopeptides comprenant l'épitope T-cytotoxique MART 27-35 pour la vaccination humaine, la capacité de ces constructions lipopeptides à induire une immunisation en l'absence d'adjuvant exogène a été testée.

Les résultats sont présentés dans la figure 4.

Plus particulièrement, l'immunogénicité des deux constructions colinéaires lipopeptidiques comprenant respectivement une ou deux chaînes palmitoyle ont été testées. Les souris ont subi une seule injection en l'absence d'adjuvant, avec chacun des lipopeptides ou encore avec le mélange d'épitope MART 27-35 et TT 830-843 utilisé comme contrôle, puis la sécrétion d'interféron-γ par les splénocytes a été mesurée après une re-stimulation *in vitro* avec le peptide MART 27-35.

Les résultats de la figure 4 indiquent que les constructions colinéaires Mp-MART et Dp-MART sont toutes deux capables d'induire in vivo une réponse cellulaire cytotoxique CD8$^+$ spécifique de l'épitope T-cytotoxique MART 27-35, en l'absence de tout adjuvant exogène.

Des résultats similaires ont été obtenus avec les cellules des ganglions lymphatiques.

Ces résultats démontrent que l'effet adjuvant des constructions lipopeptidiques colinéaires selon l'invention est suffisant pour induire une immunisation spécifique contre l'épitope T-cytotoxique, en l'absence de tout autre composé adjuvant.

**EXEMPLE 4 : Préparation d'un lipopeptide comprenant une liaison hydrazone.**

L'hydrazinopeptide et le peptide aldéhyde présentent les formules suivantes:

hydrazinopeptide : K(NH$_2$)ILKEPVHGV-OH / épitope MHCI-POL HIV-1
peptide aldéhyde : CHOCO-RTPPAYRPPNAPILK(Pam)-NH$_2$/épitope MHCII-HBVc

Le peptide aldéhyde comprend l'épitope 128-140 de la protéine core du virus de l'hépatite B (HBVc) tel que décrit par **MILICH et al.** (1998, Proc. Natl. Acad. Sci. USA, 85, 1610-1614).

16 mg (12 μmol) d'hydrazinopeptide et 15 mg (7 μmol) de peptide aldéhyde sont dissous dans 4 ml de tampon citrate/phosphate 0,01 M pH 5,4 et 1 ml de DMSO. Le pH est ajusté à 5,4 avec Na$_2$HPO$_4$ 0,2M.

Après 24h, le milieu est dilué avec 5 ml d'acide acétique et purifié sur une colonne C18 (15x500 mm). Eluant A : TFA 0,05% dans H$_2$O, Eluant B : TFA 0,05% dans CH$_3$CN/H$_2$O (80/20). Gradient 0-40%B en 10 min. puis 40-100%B en 60min. Les fractions pures sont collectées et lyophilisées. 4,9 mg de produit pur est obtenu (rendement 22%).

La formule du produit final est représentée sur la figure 5.

**EXEMPLE 5- Activité biologique des constructions lipopeptidiques selon l'invention contenant différents épitopes de HIV.**

**A)Matériels et Méthodes:**

**1. Synthèse peptidique et caractérisation:**

Des lipopeptides ont été synthétisés sur une résine MBHA (Applied Biosystems, Foster City, USA) en utilisant la stratégie classique BOC-benzyle (Merrifield, RB, 1986, Science: Volume 232 pages 341 à 343; Merrifield RB, 1963, J. Am. Chem. Soc. Vol. 85:2149-2154) et hexafluorophosphate de 2-(1H-benzotriazol-1-yl)-1,1,3,3-tetramethyluronium 0,33 M (HBTU) dans le N-méthyl-pyrrolidinone (NMP), avec une neutralisation in situ par le diisopropyléthylamine (DIPEA) (Schnolzer et al., 1992, Int. J. Pept. Protéin Res. vol.40, pages 180-193) dans un synthétiseur automatique de peptides du type Applied Biosystem 430A (Foster City, USA). Un double couplage a été systématiquement effectué en utilisant quatre équivalents d'acides aminés protégés, suivis par une étape d'acétylation systématique en utilisant de l'anhydride acétique.

Pour la synthèse des peptides dipalmitoyle , un groupe Boc-Lys(Boc)-OH (Senn Chemicals AG, Dielsdorf, CH) a été incorporé à l'extrémité N-terminale: les groupes Boc ont été simultanément éliminés par traitement classique au TFA. Pour la synthèse des peptides monopalmitoyle, un groupe Boc-Lys(Fmoc)-OH (Senn Chemicals AG, Dielsdorf, CH) a été incorporé à l'extrémité N-terminale, ce qui offrait la possibilité d'une élimination sélective du groupe Fmoc par de la pipéridine à 20% dans le NMP. Dans tous les cas, une palmitoylation sur résine a été réalisée en utilisant une activation par le HBTU en présence de DIEA, comme ci-dessus, sur le peptide par ailleurs totalement protégé. Les lipopeptides ont été ensuite déprotégés et séparés de la résine par un traitement au fluorure d'hydrogène (Tam, J.P. 1985, Int. J. Pept. protein Res, Volume 26, pages 262-273) dans un appareil Teflon-Kel F (Asti, Courbevoie, France): un protocole « High HF » a été utilisé pour les lipopeptides TT-POL1 et TT-GAG , un protocole de type « Low-High HF » a été utilisé pour les lipopeptides TT-POL2 et TT-NEF. Les lipopeptides ont été purifiés partiellement lors d'une étape de précipitation par du diéthyléther froid dans 5 ml d'une solution d'acide trifluoroacétique, puis lyophylisés. Les purifications ont été réalisées par plusieurs cycles parallèles de RP-HPLC : pour chaque cycle , 50 à 100 mg de lipopeptide brut ont été dissous dans 2ml de DMSO puis dilués dans 1 ml d'eau, avant injection sur :

- pour les monopalmitoylpeptides, une colonne de dimension 12,5 mm x 250 mm chargée avec une phase stationnaire de type C18 (0,03, 5 µm) Hyperprep Hypersil éluée à 5ml/min.;
- pour les dipalmitoylpeptides: une colonne de 12,5 mm x 250 mm chargée avec du C3(0,03, 5 µm) Zorbax et éluée à 4ml/min.

Les composés ont été élués avec un système solvant acétonitrile/H2O/0,05% de TFA à 60°C. L'homogénéité a été confirmée à l'aide d'une RP-HPLC sur deux colonnes analytiques différentes (une colonne Vydac C18 de 4,6 x 250 et une colonne C3 Zorbax de 4,6 x 150 mm): les lipopeptides étaient purs à plus de 90%. La caractérisation des peptides a été vérifiée en déterminant la composition en acides aminés après hydrolyse totale et détermination de la masse moléculaire par TOF-PDMS (Spectromètre de Mass à désorption par plasma , Bio-Ion 20, Uppsala, Suède). Les masses moléculaires mesurées ont été les suivantes: dérivés monopalmitoyle TT-GAG:[M+H]$^+$ calc., 3534, obs. 3523; TT-NEF:[M+H]$^+$ calc. 4317, obs.4318, TT-POL1:[M+H]$^+$ calc. 3534, obs. 3535; TT-POL2: [M+H]$^+$ calc. 4142, obs. 4143. Pour les dérivés dipalmitoyle TT-GAG: [M+H]$^+$ cal. 3763, obs. 3762; TT-NEF:[M+H]$^+$ calc. 4556, obs. 4553; TT-POL1:[M+H]$^+$ calc. 3773, obs. nd; TT-POL2:[M+H]$^+$ calc. 4380, obs. 4378. Tous les composés ont été obtenus sous forme de poudre lyophylisée. Les dérivés monopalmitoyle sont solubles dans l'eau. Les dérivés dipalmitoyles sont solubles dans un mélange eau-DMSO. Les rendements de purification ont été:

- pour les dérivés monopalmitoyles: TT-GAG: 16%, TT-NEF: 28%; TT-POL1: 33%; TT-POL2: 12%;
- pour les dérivés dipalmitoyles: TT-GAG: 16%; TT-NEF: 39%; TT-POL1: 22%; TT-POL2:4,5%.

Les séquences en acides aminés des peptides et lipopeptides sont présentées dans le tableau 8.

**2) Souris et immunisation**

On a utilisé des souris transgéniques HLA-A2 exprimant les domaines α1 et α2 de la molécule HLA-A2 (Vitiello et al., 1991, J. Exp. Med. Vol. 173, pages 1007-1015) . Le domaine α3 de la chaîne lourde correspond au domaine murin H-2 K$^b$. Cette caractéristique permet à la molécule de CD8$^+$ exprimée par les cellules T murines CD8$^+$ d'interagir avec le domaine α3 syngénique de la molécule MHC de classe I hybride.

Des souris transgéniques HLA-A3 ont également été utilisées.

Tous les peptides et lipopeptides ont été dissous dans une solution saline en présence de 10% de DMSO. L'induction de cellules T cytotoxiques chez les souris transgéniques HLA a été réalisée comme décrit par SETTE et al., (1994) ( J. Immunol. vol. 153, pages 5586-5592).

En résumé, des épitopes CTL (50µmg/souris) ont été mélangés avec l'épitope T-auxiliaire TT 830-843 (140 µm/souris), émulsifiés dans de l'adjuvant incomplet de Freund (IFA) et injectés à la base de la queue. Les lipopeptides ont été injectés seuls (150 µm/souris), dilués dans une solution saline sans adjuvant incomplet de Freund (IFA). Onze jours après l'injection, les souris ont été sacrifiées, et les splénocytes ainsi que les cellules des ganglions lymphatiques (LNC) ont été incubés à raison de 3 x 10$^6$/ml dans du milieu complet en présence de lymphoblastes syngéniques stimulés par le LPS irradiés, et recouverts des peptides utilisés comme cellules stimulatrices. Le milieu complet comprend du milieu RPMI 1640 (Gibco/BRL, Gaithersburg, MD), 10% de SVF, 2mM de glutamine, des antibiotiques et du β2-mercaptoéthanol (5 x $^{10}$-5M). Après six jours, les splénocytes et les cellules des ganglions lymphatiques ont été testés pour la sécrétion d'interféron-γ selon la technique ELISPOT décrite à l'exemple 3 pour les cellules de souris et la technique ELISPOT ci-dessous pour les PBMC humaines.

### 3. Test ELISPOT sur les PBMC humaines.

Des donneurs séropositifs pour HIV ont été sélectionnés pour leur expression en molecules HLA de classe I selon des données bien connues de l'homme du métier en matière de restriction vis-à-vis des peptides et lipopeptides.

Des microplaques de nitrocellulose (Millipore, Bedford, MA) ont été incubées pendant la nuit à 4°C avec 1 µg/ml d'un anticorps de capture anti-Interféron-γ humain dilué dans un tampon carbonate/bicarbonate à pH 9,6 (100 µl/puits). Après cinq lavages, les plaques ont été saturées pendant 2 heures à 37°C avec 200 µl puis lavées. Pour les cultures de cellules humaines, le milieu complet était composé de RPMI 1640, 10% de sérum albumine bovine, 2mM glutamine, d'antibiotiques et de β2-mercaptoéthanol (5 x 10$^{-5}$M). Pour le test avec le peptide, des PBMC de patients ont été ajoutés directement dans les microplaques de nitrocellulose (2 x 10$^5$ cellules/puits) et incubés pendant 24 heures à 37°C en présence de 10 mg/ml du peptide. Pour le test des lipopeptides, les PBMC ont été incubés pendant la nuit à 37 °C avec les lipopeptides, puis ont été lavés et irradiés (3000 rads). Ces cellules ont été utilisées comme cellules stimulatrices dans une co-culture (2 x 10$^5$ cellules par puits) avec des PBMC syngéniques ( rapport 1/1) dans des plaques de nitrocellulose pendant 24 heures à 37°C. Après 24 heures, les plaques contenant la culture directe (peptide) ou la co-culture (lipopeptide) ont été lavées et incubées pendant 2 heures à la température du laboratoire en présence de 100 µl d'un anticorps monoclonal anti-interféron-γ de souris biotinylé dilué à 1 µg/ml dans un tampon de PBS-Tween (0,05%) et de sérum albumine bovine (1%). Après 5 lavages dans du PBS-Tween à 0,05%, les plaques ont été incubées pendant 1 heure à la température du laboratoire en présence d'extravidine marquée à la phosphatase alcaline (Sigma, Allemagne) diluées au 1/5000 dans un tampon de PBS-Tween (0,05%) et de sérum de veau foetal (1% SVF). Les tâches (« spots ») ont été révélées en ajoutant un substrat conjugué chromogène de la phosphatase alcaline (Biorad, Hercules, CA) selon les instructions du fabriquant et les cellules formant des tâches positives pour l'Interféron-γ ont été comptées à l'aide d'une loupe binoculaire.

### 4. Test de prolifération

Les cellules des ganglions lymphatiques et les splénocytes extraits ont été cultivées dans des microplaques de 96 puits à raison de 5.10$^5$ cellules par puits dans un volume final de 200 µl de milieu complet. Les cellules ont été stimulées ou non avec 20 µg/ml d'un épitope CD4 restreint aux molécules MHC de classe II, l'épitope TT 830-843. Après 72 heures de culture à 37°C, les cellules ont été incubées pendant la nuit avec 0,25 µCi de [3H] thymidine (Amersham, Life technologie). Les cellules ont été récupérées et l'incorporation de [3H] thymidine a été analysée dans un appareil «Microbetaplate » (Wallac, Turku, Finlande).

### 5. Cellules et anticorps monoclonaux

Des cellules Jurkat-A2 (J A2/K[b]), une lignée de cellules T leucémiques humaines transfectées avec un plasmide codant la molécule hybride HLA-A2/H2K[b] (Irvin et al. 1989.

Les lymphoblastes utilisés pour stimuler les cellules effectrices *in vitro* ont été préparés de la manière suivante: des splénocytes de souris transgéniques HLA-A2 ont été cultivés avec 25µg/ml de LPS (E. Coli) et 7 µg/ml de sulfate de Dextran (Sigma). Après 72 heures à 37°C, les cellules ont été lavées et incubées avec l'épitope CD8 pendant 2 heures à 37°C. Les cellules ont été ensuite irradiées (3000 rad) , lavées trois fois et co-cultivées avec les cellules effectrices.

Les anticorps monoclonaux de capture et de détection anti-Interféron-γ de souris (clones R4-6A2 et XMG1.2, respectivement) sont commercialisés par Pharmingen (San Diego, CA). Les anticorps monoclonaux de capture et de détection anti-Interféron-γ humain (clone 1-D1K et 7-B6-1-biotine, respectivement) sont commercialisés par Mabtech (Stockholm, Suède).

**b). RESULTATS**

**1) Antigénicité des peptides et des lipopeptides chez l'homme.**

On a testé la capacité des peptides sélectionnés comprenant les épitopes d'intérêt à stimuler efficacement les lymphocytes T-cytotoxiques spécifiques de donneurs séro-positifs pour HIV . L'activation des CTL spécifiques d'épitope a été suivie en mesurant la sécrétion d'Interféron-$\gamma$ après incubation des PBMC de patients avec les peptides, à l'aide du test ELISPOT. Lorsqu'il a été établi que les peptides stimulaient efficacement les CTL spécifiques, la capacité d'activation de ces CTL spécifiques d'épitopes a été étudiée avec les lipopeptides correspondants pré-incubés avec les PBMC syngéniques.

Les résultats présentés à la figure 6a montrent que les CTLs du patient Z77 (de type HLA-2; positif pour HIV) reconnaissent les peptides POL 476-484 et GAG 77-85. Les lipopeptides TT-POL1 et TT-GAG ont également fortement stimulé ces CTLs spécifiques . Aucune différence significative d'antigénicité entre les constructions monopalmitoyle et dipalmitoyle n'a pu être observée pour ces deux lipopeptides. Par ailleurs, aucune activation des CTL spécifiques de POL 346-354 dans les PBMC du patient Z77, que ce soit avec le peptide POL 346-354 ou encore avec les lipopeptides correspondant TT-POL2 (construction monopalmitoyle et dipalmitoyle). Cependant, comme indiqué à la figure 6a, les CTLs du patient HLA-A2 Z108 reconnaissent faiblement l'épitope 346-354 et plus fortement les lipopeptides TT-POL2.

Les résultats de la figure 6b démontrent que le peptide NEF 73-82, un épitope se fixant à la fois sur les molécules HLA-A11 et HLA-A3 et formant des complexes stables avec ces molécules, induit également une forte réponse CTL spécifique dans les PBMC des patients HLA-A11 et HLA-A3 (respectivement Z 129 et P48). De plus, le lipopeptide monopalmitoyle TT-NEF a également induit une forte réponse CTL à partir de ces PBMC.

Enfin, les résultats de la figure 6c montrent que tous les peptides sélectionnés pour leur capacité de fixation sur des molécules purifiées de HLA-B35 ainsi que pour leur capacité à former des complexes stables HLA/peptide (cf figure 6c), étaient très antigéniques chez te patient HLA-B35 (P3). Les lipopeptides monopalmitoyle et dipalmitoyle TT-NEF, comprennant les peptides NEF 74-81, NEF 71-79 et NEF 68-76 étaient également antigéniques chez le patient P3.

Cependant, les lipopeptides TT-POL2 (monopalmitoyle et dipalmitoyle), bien que contenant l'épitope POL-342-350 bien reconnu, n'étaient pas antigéniques chez les patients HLA-B35 .

Les résultats de la figure 6c indiquent également que le peptide NEF 68-76 un épitope potentiel de HLA-B7, a induit efficacement une réponse CTL spécifique dans les PBMC du patient HLA-B7 Z118. De plus, les lipopeptides TT-NEF étaient aussi antigéniques chez les patients HLA-B7.

Il est important de souligner que les résultats des figures 6b et 6c montrent qu'un lipopeptide multiépitopique peut être efficacement reconnu par des individus exprimant différentes molécules HLA de classe I.

L'ensemble de ces résultats démontre clairement que les lipopeptides peuvent être efficacement transformés par les PBMC et peuvent activer les CTL spécifiques d'épitopes.

**2. Etude de l'immunogénicité des peptides et des lipopeptides dans des souris transgéniques HLA-A2 et HLA-A3.**

Le but de cette étude était de déterminer si les lipopepotides pouvaient induire la production de CTL spécifique dans des organismes naïfs, n'ayant jamais été sensibilisés avec un épitope HIV et ne possédant aucune cellule CTL spécifique d'épitope préexistante.

Les lipopeptides et peptides comprenant les épitopes d'intérêt ont été injectés à des souris transgéniques exprimant les molécules HLA-A2 ou HLA-A3. Les peptides porteurs d'épitopes T-cytotoxiques ont été co-injectés avec l'épitope T auxiliaire TT 830-843 et émulsifiés dans de l'adjuvant incomplet de Freund.

Les lipopeptides ont été dilués dans une solution saline et injectés sans adjuvant incomplet de Freund. La production de cellules CTLs spécifiques d'épitope dans ces souris a été suivie comme indiqué dans la partie Matériels et Méthodes.

Les résultats de la figure 7a montrent que les trois épitopes restreints à HLA-A2, respectivement GAG 77-85, POL 476-484 et POL 346-354, induisaient une faible réponse CTL spécifique dans les souris transgéniques HLA-A2, mais que les lipopeptides monopalmitoyle correspondant étaient capables d'induire une réponse CTL plus grande. Dans tous les cas, les lipopeptides dipalmitoyle ont induit une réponse CTL plus faible que les lipopeptides monopalmitoyle.

Les résultats de la figure 7b indiquent que l'épitope NEF-73-82 induit une forte réponse CTL spécifique dans les souris transgéniques HLA-A3. De plus, les lipopeptides monopalmitoyles TT-NEF étaient capables d'induire une réponse CTL aussi élevée que l'épitope NEF 73-82.

### 3) Etude de la nécessité d'une réponse de type T-auxiliaire pour l'induction de cellules CTLs.

Afin de déterminer l'importance de l'effet T-auxiliaire dans la production de cellules CTLs par les lipopeptides selon l'invention, des lipopeptides monopalmitoyle comprenant un épitope HIV mais dépourvus de l'épitope TT 831-843 ont été construits.

Les résultats de la figure 8a, montrent que le lipopeptide POL1 dépourvu d'épitope T-auxiliaire n'induit aucune réponse CTL spécifique, alors que le lipopeptide TT-POL1 était capable d'induire une réponse CTL spécifique beaucoup plus grande. Ce résultat indique que le peptide T auxiliaire est nécessaire afin d'induire une forte réponse CTL lorsque les lipopeptides sont injectés à la base de la queue de souris transgéniques HLA-A2 . Des expériences de prolifération lymphocytaire ont été réalisées afin de déterminer si l'épitope T-auxiliaire TT 831-843, inclu dans les lipopeptides, était capable d'induire efficacement une réponse T-auxiliaire.

Les résultats présentés à la figure 8b démontrent que les cellules de ganglions lymphatiques des souris immunisées avec le lipopeptide monopalmitoyle TT-POL1 proliféraient en réponse aux peptides TT 830-843 aussi fortement que les cellules des ganglions lymphatiques de souris immunisées avec l'épitope T-auxiliaire TT 830-843 émulsifiées dans l'adjuvant incomplet de Freund.

Ces résultats démontrent qu'une réponse de type T-auxiliaire est nécessaire afin d'obtenir une forte réponse CTL spécifique, et que la réponse T-auxiliaire est induite par l'épitope T auxiliaire contenu dans les constructions co-linéaires lipopeptidiques.

## TABLEAU 1: Epitopes de BCR-ABL

| Peptide | | Séquence | Fixation au HLA |
|---------|------|----------|-----------------|
| 247-255 | | EDAELNPRF | B44 |
| 488-496 | | SELDLEKGL | B44 |
| 768-776 | | DELEAVPNI | B44 |
| 901-934 | b2a2 | KEDALQRPV | B44 |
| 902-935 | b2a2 | EDALQRPVA | B44 |
| 986-994 | | GEKLRVLGY | B44 |
| 1176-1184 | | EDTMEVEEF | B44 |
| 1252-1260 | | MEYLEKKNF | B44 |
| 1691-1699 | | NEEAADEVF | B44 |
| 49-57 | | VNQERFRMI | B8 |
| 580-588 | | LFQKLASQL | B8 |
| 722-730 | | ARKLRHVFL | B8 |
| 786-794 | | ALKIKISQI | B8 |
| 886-893 | | CVKLQTVH | B8 |
| 928-936 | b3a2 | KALQRPVAS | B8 |
| 1830-1838 | | GAKTKATSL | B8 |
| 1975-1983 | | IQQMRNKFA | B8 |
| 1977-1984 | | QMRNKFAF | B8 |
| 252-260 | | NPRFLKDNL | B7 |
| 329-338 | | TPDCSSNENL | B7 |
| 693-701 | | TPRRQSMTV | B7 |
| 1058-1066 | | SPGQRSISL | B7 |
| 1196-1205 | | HPNLVQLLGV | B7 |
| 1560-1569 | | SPKPSNGAGV | B7 |
| 1717-1725 | | KPLRRQVTV | B7 |
| 1878-1884 | | SPAPVPSTL | B7 |
| 36-44 | | ERCKASIRR | B27 |
| 71-79 | | DRQRWGFFRR | B27 |
| 575-583 | | QRVGDLFQK | B27 |

## TABLEAU 1: Epitopes de BCR-ABL

| Peptide | | Séquence | Fixation au HLA |
|---------|---|----------|-----------------|
| 834-842 | | FRVHSRNGK | B27 |
| 642-650 | | LLYKPVDRV | A2 |
| 684-692 | | FLSSINEEI | A2 |
| 708-716 | | QLLKDSFMV | A2 |
| 714-722 | | FMVELVEGA | A2 |
| 817-825 | | KLSEQESLL | A2 |
| 881-889 | | MLTNSCVKL | A2 |
| 908-917 | | GLYGFLNVIV | A2 |
| 912-920 | | FLNVIVHSA | A2 |
| 1240-1248 | | VLLYMATQI | A2 |
| 1903-1911 | | FIPLISTRV | A2 |
| 1932-1940 | | VVLDSTEAL | A2 |
| 50-58 | | NQERFRMIY | A1 |
| 223-231 | | VGDASRPPY | A1 |
| 549-558 | | KVPELYEIHK | A3/A11 |
| 583-591 | | KLASQLGVY | A3/A11 |
| 715-724 | | MVELVEGARK | A3/A11 |
| 916-923 | | IVHSATGFK | A3/A11 |
| 920-928 | b3a2 | ATGFKQSSK | A3/A11 |
| 924-932 | b3a2 | KQSSKALQR | A3/A11 |
| 1156-1165 | | EVYEGVWKKY | A3/A11 |
| 1311-1320 | | SLAYNKFSIK | A3/A11 |
| 1499-1509 | | NLFSALIKK | A3/A11 |
| 1724-1734 | | TVAPASGLPHK | A3/A11 |
| 1905-1914 | | LISTRVSLRK | A3/A11 |
| 1922-1930 | | RIASGAITK | A3/A11 |

## TABLEAU 2 - Epitopes de la p53

- épitopes de la p53 se liant au HLA-A1:
RVEGNLARVEY (196-205)
GSDCTTIHY (226-234)
- épitopes de la p53 se liant au HLA-A2:
LLPENNVLSPL (25-35)
RMPEAAPPV (65-73)
RMPEAAPRV
ALNKMFCQL (129-137)
STPPPGTRV (149-157)
GLAPPQHLIRV (187-197)
LLGRNSFEV (264-272)
PLDGEYFTL (322-330)
- épitopes de la p53 se liant au HLA-A3:
RVRAMAIYK (156-164)
RRTEEENLR (282-290)
ELPPGSTKR (298-306)
- épitopes de la p53 se liant au HLA-B7:
LPENNVLSPL (26-35)
APRMPEAAPPV (63-73)
APRMPEAAPRV
APPQHLIRV (189-197)
RPILTIITL (249-257)
KPLDGETYFTL (321-330)
- épitopes de la p53 se liant au HLA-B8:
CQLAKTCPV (135-143)
GLAPPQHLI (187-195)
NTFRHSVVV (210-218)
- épitopes de la p53 se liant au HLA-B51:
LLPENNVLSPL (25-35)
RMPEAAPPV (65-73)
LIRVEGNLRV (194-203)

## TABLEAU 3
## Epitopes des protéines E$_6$ et E$_7$

YMLDLQPETT (E7 11-20)

LLMGTLGIV (E7 82-90)

TLGIVCPI (E7 86-93)

TIHDIILECV (E6 29-38)

KLPQLCTEL (E6 18-26)

RPPKLPQL (E6 8-15)

LRREVYDFAFRDLCIVYRDGNPY (E6 45-67)

ISEYRHYCY (E6 80-88)

EKQRHLDKKQRFHNIRGRWT (E6 121-140)

GQAEPDRAHYNIVTF (E7 43-57)

QAEPDRAHY (E7 44-52)

EPDRAHYNIV (E7 46-55)

## TABLEAU 4: Epitopes du virus VIH-1

HLA-A1

(Nef 96-106: GLEGLIHSQRR
(Nef 121-128: FPDWQNYT
(Nef 137-145: LTFGWCYKL
(Nef 184-191: RFDSRLAF
(Nef 195-202: ARELHPEY :

HLA-A2

Gp120 121-129: KLTPLCVTL
P17 77-85: SLYNTVATL
RT 200-208: ALVEICTEM
RT 275-285: VLDVGDAYFSV
RT 346-354: KIYQYMDDL ·
RT 368-376: KIEELRQHL
RT 376-387: LLRWGLTTPDK
RT 476-484: ILKEPVHGV·
RT 588-596: PLVKLWYQL
RT 683-692: ELVNQDEQL·
Nef 136-145: PLTFGWCFKL
Nef 180-189: VLQWRFDSRL
Nef 190-198: ALHHVAREL
Gp41 818-826: SLLNATVDI
P24 185-193: DLNTMLNTV
RT 346-354: VIYQYMDDL
RT 588-596: PLVKLWYQL
Pro 143-152: VLVGPTPVNI
(Gp120 37-44: TVYYGVPV
(Gp120 115-122: SLKPCVKL
(Gp120 313-321: RIQRGPGRA
(Gp120 197-205: TLTSCNTSV
(Gp120 428-435: FINMWQEV
(Gp41 836-844: VVQGAYRAI
(p24 219-228: HAGPIAPGQM
(p15 422-431: QMKDCTERQA
(p15 448-456: FLQSRPETA
(RT 681-691: ESELVNQIIEG

## TABLEAU 4: Epitopes du virus VIH-1 (suite)

HLA-A3

P17 18-26: KIRLRPGGK ·
P17 20-28: RLRPGGKKK ·
RT 200-210: ALVEICTEMEK
RT 325-333: AIFQSSMTK
RT 359-368: DLEIGQHRTK
Nef 73-82: QVPLRPMTYK ·
Gp120 37-46: TVYYGVPVWK
Gp41 775-785: RLRDLLLIVTR
P17 18-26: KIRLRPGGK

HLA-A11

RT 325-333: AIFQSSMTK
RT 507-517: QIYQEPFKNLK
Nef 73-82: QVPLRPMTYK ·
Nef 84-92: AVDLSHFLK ·
p24 349-359: ACQVGGPGHK
P17 83-91: ATLYCVHQR ·

HLA-A24 (A9)

Gp120 52-61: LFCASDAKAY
Gp41 591-598: YLKDQQLL ·
ou 590-597: RYLKDQQLL
(RT 484-492: VYYDPSKDL
(RT 508-516: IYQEPFKNL
(RT 681-691: ESELVNQIIEG

HLA-A25 (A10)
P24 203-212: ETINEEAAEW
HLA-A26 (A10)
P24 167-175: EVIPMFSAL ·

HLA-A30 (A19)
(Gp41 845-852: RAIRHIPRR

HLA-A31 (A19)
Gp41 775-785: RLRDLLLIVTR

HLA-A32 (A19)
Gp120 424-432: RIKQIINMW
Gp41 774-785: HRLRDLLLI ·
RT 559-568: PIQKETWETW

## TABLEAU 4: Epitopes du virus VIH-1 (Suite)

HLA-A33 (A19)
           (P24 266-275: IILGLNKIVR

HLA-B7
               RT 699-707: YLAWVPAHK
               Nef 6S-77: FPVTQVPLR ·
               Nef 12S-137: TPGPGVRYPL
               Gp120 303-312: RPNNNTRKSI
               Gp41 848-856: IPRRIRQGL
               RT 699-707: YLAWVPAHK

HLA-B8
               Gp120 2-10: RVKEKYQHL
               P17 24-32: GGKKKYKLK
               Nef 90-97: FLKEKGGL =
               P24 259-267: GEIYKRWII
               Gp41 591-598: YLKDQQLL
               (Gp41 849-856: PRRIRQGL
                 ou 851-859: RIRQGLERIL
               (P24 329-337: DCKTILKAL
               (RT 185-193: GPKVKQWPL
               (Nef 182-189: EWRFDSRL ·

HLA-B14
               Gp41 589-597: ERYLKDQQL
               P24 298-306: DRFYKTLRA ·
               (P24 183-191 ? : DLNTMLNTV
               (p24 304-313: LRAEQASVQEV
               (p24 305-313: RAEQASVQEV

HLA-B18
               Nef 135-143: YPLTFGWCY
               Nef 135-143: YPLTFGWCF

## TABLEAU 4: Epitopes du virus VIH-1

### HLA-B27

P24 263-272: KRWIILGLNK
Nef 73-82: QVPLRPMTYK
Nef 134-141: RYPLTFGW
ou 133-141: YPLTFGW
Gp41 589-597: ERYLKDQQL
(Gp41 791-800: GRRGWEALKY

### HLA-B35

Gp120 78-86: DPNPQEVVL ·
Gp120 257-265: RPVVSTQLL
RT 285-294: VPLDKDFRKY ·
RT 323-331: SPAIFQSSM ·
RT 342-350: NPDIVIYQY (consensus clade B)
RT 460-468: IPLTEEAEL - ·
RT 598-608: EPIVGAETFY
Nef 68-76: FPVRPQVPL ·
Nef 74-81: VPLRPMTY ·
Gp41 611-619: TAVPWNASW
Gp120 42-52: VPVWKEATTTL · ·
P17 124-132: NSSQVSQNY (consensus clade B)
P24 254-262: PPIPVGEIY (consensus clade B)

### HLA-B37

Nef 120-128: YFPDWQNYT

### HLA-B44 (B12)

P24 178-186: SEGATPQDL ·
(p24 175-184: LESGATPQDL

22

## TABLEAU 4: Epitopes du virus VIH-1

### HLA-B51 (B5)

gp41 562-570: RAIEAQQHL
RT 200-208: ALVEICTEM ·
RT 209-217: EKEGKISKI
RT 295-302: TAFTIPSI ·

### HLA-B52 (B5)

Nef 190-198: AFHHVAREL

### HLA-B55 (B22)

Gp120 42-51: VPVWKEATTTL

### HLA-B57 et B58 (B17)

P24 240-249: TSLTQEQIGW ·
Nef 116-125: HTQGYFPDWQ
ou 116-124: HTQGYFPDW ·
Nef 120-128: YFPDWQA
(P24 147-155: ISPRTLNAW
(P24 164-172: FSPEVIPMF

### HLA-Bw62 (B15)

P17 20-29: RLRPGGKKKY ·
P24 268-277: LGLNKIVRMY
RT 427-438: LVGKLNWASQIY
Nef 84-91: AVDLSHFL ·
Nef 117-127: TQGYFPDWQNY

### HLA-Cw4

gp120 380-388: SFNCGGEFF

### HLA-Cw8

RT 663-672: VTDSQYALGI ·
P24 305-313: RAEQASQEV
Nef 82-91: KAALDLSHPL

### HLA-Cw?

P24 308-316: QATQEVKNW

## TABLEAU 5 - Epitopes de Mélanome humain

| Gene/protéine | MHC restriction | Peptide | Position des acides aminés |
|---|---|---|---|
| Tyrosinase | HLA-A2 | MLLAVLYCL | 1-9 |
| | HLA-A2 | YMNGTMSQV | 369-377 |
| | | YMDGTMSQV | |
| | HLA-A24 | AFLPWHRLF | 206-214 |
| | HLA-B44 | SEIWRDIDF | 192-200 |
| Pmel17$^{gp100}$ | HLA-A2 | KTWGQYWQV | 154-162 |
| | HLA-A2 | AMLGTHTMEV | 177-186 |
| | HLA-A2 | MLGTHTMEV | 178-186 |
| | HLA-A2 | ITDQVPFSV | 209-217 |
| | HLA-A2 | YLEPGPVTA | 280-288 |
| | HLA-A2 | LLDGTATLRL | 457-466 |
| | HLA-A2 | VLYRYGSFSV | 476-485 |
| | HLA-A2 | SLADTNSLAV | 570-579 |
| | HLA-A3 | ALLAVGATK | 17-25 |
| Melan-A$^{MART-1}$ | HLA-A2 | (E)AAGIGILTV | 26(7)-35 |
| | HLA-A2 | ILTVILGVL | 32-40 |
| gp$^{75TRP-1}$ | HLA-A31 | MSLQRQFLR | |
| TRP-2 | HLA-A31 | LLGPGRPYR | 197-205 |

## TABLEAU 6: Epitopes de tumeurs résultant de mutations

| Gene/proteine | Tumeur | MHC Restriction | Peptide | Position des acides aminés |
|---|---|---|---|---|
| MUM-1 | Mélanome | HLA-B44 | EEKLIVVLF | 30-38 |
| CDK4 | Mélanome | HLA-A2 | ACDPHSGHFV | 23-32 |
| β-catenine | Mélanome | HLA-A24 | SYLDSGIHF | 29-37 |
| CASP-8 | carcinome squameux de la tête et du cou | HLA-B35 | FPSDSWCYF | 476-484 |

## TABLEAU 7

## Antigènes communs à diverses tumeurs

| Gene | tissu où a lieu l'expression normale | MHC restriction | Peptide antigénique | Position des acides aminés |
|------|------|------|------|------|
| MAGE-1 | testicules | HLA-A1 | EADPTGHSY | 161-169 |
|  |  | HLA-Cw16 | SAYGEPRKL | 230-238 |
| MAGE-3 | testicules | HLA-A1 | EVDPIGHLY | 168-176 |
|  |  | HLA-A2 | FLWGPRALV | 271-279 |
|  |  | HLA-B44 | MEVDPIGHLY | 167-176 |
| BAGE | testicules | HLA-Cw16 | AARAVFLAL | 2-10 |
| GAGE-1/2 | testicules | HLA-Cw6 | YRPRPRRY | 9-16 |
| RAGE-1 | rétine | HLA-B7 | SPSSNRIRNT | 11-20 |
| GnTV | aucun | HLA-A2 | VLPDVFIRC | 38-64 |

**TABLEAU 8 : Peptides et lipopeptides utilisés**

| Désignation | Position | Séquences d'acides aminés | MHC-I restriction | Référence |
|---|---|---|---|---|
| peptides HIV | | | | |
| GAG | 77-85 | SLYNTVATL | HLA-A2.01 | (6) |
| POL | 476-484 | ILKEPVHGV | HLA-A2.01 | (5) |
| POL | 346-354 | VIYQYMDDL | HLA-A2.01 | (2) |
| POL | 342-350 | NPDIVIYQY | HLA-B35 | (4) |
| POL | 343-350 | PDIVIYQY | HLA-B44 | non décrit |
| POL | 347-354 | FYQYMDDL | HLA-A24 | non décrit |
| POL | 345-354 | IVIYQYMDDL | HLA-A2 | non décrit |
| NEF | 68-76 | FPVTPQVPL | HLA-B7-B35 | (3) |
| NEF | 71-79 | TPQVPLRPM* | HLA-B35 | non décrit |
| NEF | 74-81 | VPLRPMTY | HLA-B35 | (1) |
| NEF | 73-82 | QVPLRPMTYK | HLA-A3, -A11 | (1) |
| | | | | |
| Lipopeptides HIV | | | | |
| TT-GAG | 77-85 | Palm-K-GR QYIKANSKFIGITE RGR SLYNTVATL | HLA-A2.01 | |
| TT-POL1 | 476-484 | Palm-K-GR QYIKANSKFIGITE RGR ILKEPVHGV | HLA-A2.01 | |
| TT-POL2 | multiépitopic | Palm-K-GR QYIKANSKFIGITE RGR NPDI VIYQYMDDL | multi HLA | |
| TT-NEF | multiépitopic | Palm-K-GR QYIKANSKFIGITE RGR FPVTP QVPLRPMTYK | multi HLA | |
| | | | | |
| Peptide T auxiliaire | | | | |
| Toxine tétanique | 830-843 | QYIKANSKFIGITE | HLA-DR, H-2 K$^b$ | |

REFERENCES DU TABLEAU 8:

- **CULMANN-PENCIOLELLI, B., S. LAMHAMEDI-CHERRADI, I. COUILLIN, N. GUEGAN, J.P. LEVY, J.G. GUILLET, and E. GOMARD. 1994.** Identification of multirestricted immunodominant regions recognized by cytolytic T lymphocytes in the human immunodeficiency virus type 1 Nef protein [published erratum appears in J. Virol. 1995 Jan: 69 (1):618]. J. Virol. 68:p7336-43.

- **HARRER, E., T. HARRER, P. BARBOSA, M. FEINBERG, R.P. JOHNSON, S. BUCHBINDER, and B.D. WALKER. 1996.** Recognition of the highly conserved YMDD region in the human immunodeficiency virus type 1 reverse transcriptase by HLA-A2- restricted cytotoxic T lymphocytes from an asymptomatic long-term nonprogressor. J. Infect Dis. 173: p476-9.

- **LUCCHIARI-HARTZ, M., M. BAUER, G. NIEDERMANN, B. MAIER, A. MEYERHANS, and K. ELCHMANN. 1996.** Human immune response to HIV-1 Nef. II. Induction of HIV-1/HIV-2 Nef cross-reactive cytotoxic T lymphocytes in peripheral blood lymphocytes of non-infected healthy individuals. Int Immunol. 8:p577-84.

- **ROWLAND-JONES, S., J. SUTTON, K. ARIYOSHI, T. DONG, F. GOTCH, S. McADAM, D. WHITBY, S. SABALLY, A. GALLIMORE, T. CORRAH et al. 1995.** HIV specific cytotoxic T-cells in HIV-exposed but uninfected Gambian women. Nat Med. 1:59-64.

- **TSOMIDES, T.J. B.D. WALKER, and H.N. EISEN. 1991.** An optimal viral peptide recognized by CD8+ T cells binds very tightly to the restricting class I major histocompatibility complex protein on intact cells but not to the purified class I protein. Proc. Natl Acad. Sci USA. 88:p11276-80.

- **WALKER, B.D. C. FLEXNER, K. BIRCH-LIMBERGER, L. FISHER, T.J. PARADIS, A. ALDOVINI, R. YOUNG, B. MOSS, and R. T.SCHOOLEY. 1989.** Long-term culture and fine specificity of human cytotoxic T-lymphocyte clones reactive with human immunodeficiency virus type 1. Proc Natl acad Sci USA. 86:p9514-8.

## Revendications

1. Lipopeptide comprenant au moins un épitope T auxiliaire, au moins un épitope CTL et au moins un résidu lipidique, **caractérisé en ce que** les épitopes et la partie lipidique d'une part, et les épitopes d'autre part, sont séparés indépendamment par des séquences d'acides aminés, appelées espaceurs, au moins l'un des espaceurs comprenant des enchaînements de 1 à 10 acides aminés choisis parmi les acides aminés glycine, arginine, acide glutamique et acide aspartique.

2. Lipopeptide selon la revendication 1, **caractérisé en ce qu'**au moins l'un des espaceurs comprend entre 1 et 10, glycines et/ou arginines.

3. Lipopeptide selon l'une des revendications 1 à 2, **caractérisé en ce qu'**au moins l'un des espaceurs présente l'une des séquences suivantes:

### GLY ARG ou ARG GLY ARG.

4. Lipopeptide selon l'une des revendications 1 à 3 **caractérisé en ce qu'**au moins l'un des espaceurs comprend un ou plusieurs acides glutamique et/ou acides aspartique.

5. Lipopeptide selon l'une des revendications 1 à 3, **caractérisé en ce que** les espaceurs intègrent une cystéine ou une chaîne alkyle fonctionnalisée par un groupe thiol et des liaisons non-peptidiques.

6. Lipopeptide selon l'une quelconque des revendications 1 à 3, **caractérisé en ce que** les espaceurs comprennent un groupe choisi parmi un groupe thiazolidine, oxime ou hydrazone.

7. Lipopeptide selon l'une des revendications 1 à 6, **caractérisé en ce qu'**il comprend l'enchaînement :

   - d'une partie lipidique,
   - d'un premier espaceur,
   - d'un épitope T auxiliaire,
   - d'un second espaceur, et
   - d'un épitope CTL.

8. Lipopeptide selon l'une des revendications 1 à 6, **caractérisé en ce qu'**il comprend l'enchaînement:

- d'une partie lipidique,
- d'un premier espaceur,
- d'un épitope T auxiliaire,
- d'un second espaceur,
- d'un premier épitope CTL,
- d'un troisième espaceur, et
- d'un second épitope CTL.

**9.** Lipopeptide selon l'une des revendications 1 à 8, **caractérisé en ce que** la partie lipidique comprend une ou plusieurs chaînes dérivées d'acides gras ou d'alcools gras en $C_{10}$ à $C_{20}$, linéaires ou ramifiées, saturées ou insaturées, ou un dérivé de stéroïde.

**10.** Lipopeptide selon l'une des revendications 1 à 9, **caractérisé en ce que** la partie lipidique comprend au moins deux chaînes dérivées d'acides gras ou d'alcools gras en $C_{10}$ à $C_{20}$, liées entre elles par un ou plusieurs acides aminés.

**11.** Lipopeptide selon l'une des revendications 1 à 10, **caractérisé en ce que** la partie lipidique est constituée de deux chaînes d'acide palmitique liées aux groupements $NH_2$ d'une lysine.

**12.** Lipopeptide selon l'une des revendications 1 à 10, **caractérisé en ce que** la partie lipidique comprend une ou plusieurs chaînes choisies parmi l'acide palmitique, l'acide oléique, l'acide linoléique, l'acide linolénique, l'acide 2-amino hexadécanoïque, le pimélautide ou le triméxautide.

**13.** Lipopeptide selon l'une des revendications 1 à 12, **caractérisé en ce que** la partie non lipidique comprend entre 15 et 100 acides aminés.

**14.** Lipopeptide selon l'une des revendications 1 à 13, **caractérisé en ce que** l'épitope T auxiliaire est un épitope multivalent.

**15.** Lipopeptide selon l'une des revendications 1 à 14, **caractérisé en ce que** l'épitope T auxiliaire est le peptide 830-843 de la toxine tétanique présentant la séquence suivante:

## QYIKANSKFIGITE

**16.** Lipopeptide selon l'une des revendications 1 à 14, **caractérisé en ce que** l'épitope T auxiliaire est l'épitope de l'hémagglutinine du virus de l'influenza ou l'épitope PADRE.

**17.** Lipopeptide selon l'une des revendications 1 à 16, **caractérisé en ce qu'**il contient au moins un épitope CTL d'une protéine spécifique du mélanome, d'une protéine du VIH, du VHB, du papillomavirus, de la protéine p-53 ou d'une protéine du stade intra-hépatocytaire de *Plasmodium Falciparum*.

**18.** Utilisation d'un lipopeptide selon l'une des revendications 1 à 17 pour la fabrication d'un médicament ou d'un vaccin pour l'induction d'une réponse immunitaire spécifique.

**19.** Utilisation d'un lipopeptide selon l'une des revendications 1 à 17 pour la fabrication d'un médicament pour l'induction d'une réponse immunitaire à l'encontre du VIH, du VHB, du papillomavirus, de la p-53 du mélanome, ou de la malaria.

**20.** Composition pharmaceutique, **caractérisée en ce qu'**elle contient une dose pharmacologiquement efficace d'un ou plusieurs lipopeptides selon l'une des revendications 1 à 17 et des excipients pharmaceutiquement compatibles.

**21.** Médicament ou vaccin, **caractérisé en ce qu'**il contient un ou plusieurs lipopeptides selon l'une des revendications 1 à 17.

**Patentansprüche**

1. Lipopeptid, das wenigstens ein Helfer-T-Epitop, wenigstens ein CTL-Epitop und wenigstens einen Lipidrest umfasst, **dadurch gekennzeichnet, dass** die Epitope des Lipidteils einerseits und die Epitope andererseits unabhängig durch Aminosäuresequenzen, die Spacer genannt werden, voneinander getrennt sind, wobei wenigstens einer der Spacer Verkettungen von 1 bis 10 Aminosäuren umfasst, die aus den Aminosäuren Glycin, Arginin, Glutaminsäure und Asparaginsäure ausgewählt sind.

2. Lipopeptid gemäß Anspruch 1, **dadurch gekennzeichnet, dass** wenigstens einer der Spacer zwischen 1 und 10 Glycin- und/oder Argininreste umfasst.

3. Lipopeptid gemäß einem der Ansprüche 1 bis 2, **dadurch gekennzeichnet, dass** wenigstens einer der Spacer eine der folgenden Sequenzen aufweist:

## Gly-Arg oder Arg-Gly-Arg.

4. Lipopeptid gemäß einem der Ansprüche 1 bis 3, **dadurch gekennzeichnet, dass** wenigstens einer der Spacer einen oder mehrere Glutaminsäure- und/oder Asparaginsäurereste umfasst.

5. Lipopeptid gemäß einem der Ansprüche 1 bis 3, **dadurch gekennzeichnet, dass** die Spacer einen Cysteinrest oder eine durch eine Thiolgruppe funktionalisierte Alkylkette und nichtpeptidische Bindungen beinhaltet.

6. Lipopeptid gemäß einem der Ansprüche 1 bis 3, **dadurch gekennzeichnet, dass** die Spacer eine Gruppe umfassen, die aus einer Thiazolidin-, Oxim- oder Hydrazongruppe ausgewählt ist.

7. Lipopeptid gemäß einem der Ansprüche 1 bis 6, **dadurch gekennzeichnet, dass** es die Verkettung

- eines Lipidteils,
- eines ersten Spacers,
- eines T-Helfer-Epitops,
- eines zweiten Spacers und
- eines CTL-Epitops

umfasst.

8. Lipopeptid gemäß einem der Ansprüche 1 bis 6, **dadurch gekennzeichnet, dass** es die Verkettung

- eines Lipidteils,
- eines ersten Spacers,
- eines T-Helfer-Epitops,
- eines zweiten Spacers,
- eines ersten CTL-Epitops,
- eines dritten Spacers und
- eines zweiten CTL-Epitops,

umfasst.

9. Lipopeptid gemäß einem der Ansprüche 1 bis 8, **dadurch gekennzeichnet, dass** der Lipidteil eine oder mehrere Ketten, die von linearen oder verzweigten, gesättigten oder ungesättigten $C_{10}$- bis $C_{20}$-Fettsäuren oder -Fettalkoholen abgeleitet sind, oder ein Steroidderivat umfasst.

10. Lipopeptid gemäß einem der Ansprüche 1 bis 9, **dadurch gekennzeichnet, dass** der Lipidteil wenigstens zwei Ketten umfasst, die von $C_{10}$- bis $C_{20}$-Fettsäuren oder -Fettalkoholen abgeleitet sind und durch eine oder mehrere Aminosäuren miteinander verbunden sind.

11. Lipopeptid gemäß einem der Ansprüche 1 bis 10, **dadurch gekennzeichnet, dass** der Lipidteil aus zwei Palmitinsäureketten besteht, die mit den $NH_2$-Gruppen eines Lysinrests verbunden sind.

12. Lipopeptid gemäß einem der Ansprüche 1 bis 10, **dadurch gekennzeichnet, dass** der Lipidteil eine oder mehrere Ketten umfasst, die aus Palmitinsäure, Oleinsäure, Linolsäure, Linolensäure, 2-Aminohexadecansäure, Pimelautid oder Trimexautid ausgewählt sind.

13. Lipopeptid gemäß einem der Ansprüche 1 bis 12, **dadurch gekennzeichnet, dass** der Nichtlipidteil zwischen 15 und 100 Aminosäuren umfasst.

14. Lipopeptid gemäß einem der Ansprüche 1 bis 13, **dadurch gekennzeichnet, dass** das T-Helfer-Epitop ein mehrwertiges Epitop ist.

15. Lipopeptid gemäß einem der Ansprüche 1 bis 14, **dadurch gekennzeichnet, dass** es sich bei dem T-Helfer-Epitop um das Peptid 830-843 des Tetanustoxins mit der folgenden Sequenz handelt:

### QYIKANSKFIGITE

16. Lipopeptid gemäß einem der Ansprüche 1 bis 14, **dadurch gekennzeichnet, dass** es sich bei dem T-Helfer-Epitop um das Epitop des Hämagglutinins des Influenzavirus oder das Epitop PADRE handelt.

17. Lipopeptid gemäß einem der Ansprüche 1 bis 16, **dadurch gekennzeichnet, dass** es wenigstens ein CTL-Epitop eines spezifischen Melanomproteins, eines Proteins von HIV, HBV, Papillomvirus, eines p53-Proteins oder eines Proteins des intrahepatocytären Stadiums von *Plasmodium falciparum* enthält.

18. Verwendung eines Lipopeptids gemäß einem der Ansprüche 1 bis 17 zur Herstellung eines Medikaments oder eines Impfstoffs für die Induktion einer spezifischen Immunantwort.

19. Verwendung eines Lipopeptids gemäß einem der Ansprüche 1 bis 17 zur Herstellung eines Medikaments für die Induktion einer Immunantwort gegen HIV, HBV, Papillomvirus, Melanom-p53 oder Malaria.

20. Pharmazeutische Zusammensetzung, **dadurch gekennzeichnet, dass** sie eine pharmakologisch wirksame Dosis eines oder mehrerer Lipopeptide gemäß einem der Ansprüche 1 bis 17 und pharmazeutisch verträgliche Trägersubstanzen enthält.

21. Medikament oder Impfstoff, **dadurch gekennzeichnet, dass** es bzw. er ein oder mehrere Lipopeptide gemäß einem der Ansprüche 1 bis 17 enthält.

**Claims**

1. A lipopeptide comprising at least one auxiliary T epitope, at least one CTL epitope and at least one lipid moiety **characterized in that** the epitopes and the lipid portion on the one hand and the epitopes on the other hand are independently separated by amino-acid sequences, so-called spacers, at least one of the spacers having linkages with 1 to 10 amino-acids selected from glycine, arginine, glutamic acid and aspartic acid.

2. A lipopeptide according to claim 1, **characterized in that** at least one of the spacers comprises from 1 to 10 glycines and/or arginines.

3. A lipopeptide according to one of claims 1 to 2, **characterized in that** at least one of the spacers has one of the following sequences:

### GLY ARG

or

## ARG GLY ARG

**4.** A lipopeptide according to one of claims 1 to 3, **characterized in that** at least one of the spacers comprises one or more glutamic and/or aspartic acids.

**5.** A lipopeptide according to one of claims 1 to 3, **characterized in that** the spacers comprise a cysteine or an alkyl chain functionalized by a thiol group and non peptide bonds.

**6.** A lipopeptide according to any one of claims 1 to 3, **characterized in that** the spacers comprise a group selected from a thiazolidine, oxime or hydrazone group.

**7.** A lipopeptide according to one of claims 1 to 6, **characterized in that** it comprises the linkage of :

- a lipid portion,
- a first spacer,
- an auxiliary T epitope,
- a second spacer, and
- a CTL epitope.

**8.** A lipopeptide according to one of claims 1 to 6, **characterized in that** it comprises the linkage of :

- a lipid portion,
- a first spacer,
- an auxiliary T epitope,
- a second spacer,
- a first CTL epitope,
- a third spacer, and
- a second CTL epitope.

**9.** A lipopeptide according to one of claims 1 to 8, **characterized in that** the lipid portion comprises one or more linear or branched, saturated or insaturated chains derived from $C_{10}$-$C_{20}$ fatty acids or alcohols, or a steroid derivative.

**10.** A lipopeptide according to one of claims 1 to 9, **characterized in that** the lipid portion comprises at least two chains derived from $C_{10}$-$C_{20}$ fatty acids or alcohols, bonded together by one or more amino-acids.

**11.** A lipopeptide according to one of claims 1 to 10, **characterized in that** the lipid portion is made of two palmitic acid chains linked to the $NH_2$ groups of a lysine.

**12.** A lipopeptide according to one of claims 1 to 10, **characterized in that** the lipid portion comprises one or more chains selected from palmitic, oleic, linoleic, linolenic, 2-amino-hexadecanoic acids, pimelautide or trimexautide.

**13.** A lipopeptide according to one of claims 1 to 12, **characterized in that** the non lipid portion comprises from 15 to 100 amino-acids.

**14.** A lipopeptide according to one of claims 1 to 13, **characterized in that** the auxiliary T epitope is a multivalent epitope.

**15.** A lipopeptide according to one of claims 1 to 14, **characterized in that** the auxiliary T epitope is the 830-843 peptide of the tetanic toxin having the following sequence:

## QYIKANSKFIGITE

**16.** A lipopeptide according to one of claims 1 to 14, **characterized in that** the auxiliary T epitope is the haemagglutinin epitope of the influenza virus or the PADRE epitope.

**17.** A lipopeptide according to one of claims 1 to 16, **characterized in that** it comprises at least a CTL epitope of a protein specific of the melanoma, a HIV protein, VBH, papillomavirus, protein p53 or a protein from the intrahepatocytary stadium of *Plasmodium falciparum*.

**18.** Use of a lipopeptide according to one of claims 1 to 17 for making a medicine or a vaccine for the induction of a specific immune response.

**19.** Use of a lipopeptide according to one of claims 1 to 17 for making a medicine for the induction of an immune response against HIV, VBH, papillomavirus, the melanoma p-53 or malaria.

**20.** A pharmaceutical composition, **characterized in that** it comprises a pharmacologically efficient dose of one or more lipopeptides according to one of claims 1 to 17 and pharmaceutically compatible excipients.

**21.** A medicine or vaccine, **characterized in that** it comprises one or more lipopeptides according to one of claims 1 to 17.

FIGURE 1

**FIGURE 2A**

**FIGURE 2B**

EP 1 068 226 B1

Figure 3a

Figure 3b

**Fig.** 4

EP 1 068 226 B1

**y axis:** g-IFN SFC / 10.6 Cellules

2500

2000

1500

1000

500

0

**x axis categories:** MART 27-35 + TT    Mp-MART    Dp-MART

**x axis label:** Immunisation

**Legend:**
□ O peptide
▨ MART 27-35

FIGURE 5

# HLA-A2

Z 77

Chart (top):
- Aucun peptide
- TT-NEF
- TT-POL di
- TT-POL mono
- POL 476-484
- TT-GAG di
- TT-GAG Mono
- GAG 77-85

x-axis: 0    500    1000    1500

Z 108

Chart (bottom):
- TT-RT di
- TT-RT mono
- RT 346-354
- Aucun peptide

x-axis: 0    50    100    150    200    250

Nombre de taches par $10^6$ cellules

Figure 6a

## HLA-A11

Z 129

## HLA-A3

P 48

Nombre de taches par $10^6$ cellules

Figure 6b

# HLA-B35

**P 3**

# HLA-B7

**Z 118**

Nombre de taches par $10^6$ cellules

Figure 6c

Nombre de taches par $10^6$ cellules

Figure 7a

Nombre de taches par $10^6$ cellules

Figure 7b

■ Jurkat

▨ Jurkat + POL 476-484

Nombre de taches par $10^6$ cellules

Figure 8a

Incorporation de $^3$H thymidine (x $10^{-3}$ cpm)

Figure 8b